# EUROPEAN PATENT SPECIFICATION

(11) **EP 3 002 290 B1**
(45) Date of publication and mention of the grant of the patent: **09.09.2020**
(21) Application number: 15173458.9
(22) Date of filing: 18.06.2008
(51) Int. Cl.: A61Q 19/06, A61K 8/63, C07J 1/00, C07J 9/00, C07J 13/00

(54) **SYNTHESIS OF DEOXYCHOLIC ACID (DCA)**
SYNTHESE VON DESOXYCHOLSAEURE (DCA)
SYNTHESE D' ACIDE DESOXYCHOLIQUE (DCA)

(30) Priority: 19.06.2007 US 945035 P; 20.08.2007 US 956875 P; 21.02.2008 US 35339; 25.04.2008 GB 0807615; 16.05.2008 US 153446
(43) Date of publication of application: 06.04.2016
(62) Divisional of application: 11181831.6
(73) Proprietor: Allergan Sales, LLC, Madison, NJ 07940 (US)
(72) Inventor: MORIARTY, Robert M., Michiana Shores, IN Indiana 46360-1126 (US); DAVID, Nathaniel E., San Francisco, CA 94115 (US); MAHMOOD, Nadir Ahmeduddin, Calabasas, CA California 91302 (US); PRASAD, Achampeta Rathan, 500 007 Hyderabad (IN); SWARINGEN, Roy A., Jr., Durham, NC North Carolina 27712 (US); REID, John Gregory, Groton, MA Massachusetts 01450 (US); SAHOO, Akhila Kumar, 751006 Orissa (IN)
(74) Representative: Hoffmann Eitle

(56) References cited:
- RUZENA MICKOVA: "Reduction of 12-oxo derivatives of some bile acids", COLLECTION OF CZECHOSLOVAK CHEMICAL COMMUNICATIONS, INSTITUTE OF ORGANIC CHEMISTRY & BIOCHEMISTRY, PRAGUE; CZ, vol. 50, 1 January 1985 (1985-01-01), pages 1239-1243, XP009169299, ISSN: 0010-0765
- REICHSTEIN T. ET AL.: "Über Gallensäuren und verwandte Stoffe. 12. Mitteilung. Vereinfachte präparative Herstellung reiner Desoxy-cholsäure und eigener ihrer Derivate", HELVETICA CHIMICA ACTA, vol. 25, no. 5, 24 October 2004 (2004-10-24), pages 797-805, XP002509789, DOI: 10.1002/hlca.19430260702
- B. KOECHLIN ET AL: "Über Gallensäuren und verwandte Stoffe. 16. Mitteilung. 3[alpha],12[alpha]-Dioxy-cholansäure (12-epi-Desoxycholsäure)", HELVETICA CHIMICA ACTA, vol. 25, no. 5, 1 August 1942 (1942-08-01) , pages 918-935, XP055061610, CH ISSN: 0018-019X, DOI: 10.1002/hlca.19420250516
- LOUIS F. FIESER ET AL: "Oxidation of Steroids. III. Selective Oxidations and Acylations in the Bile Acid Series", JOURNAL OF THE AMERICAN CHEMICAL SOCIETY, vol. 72, no. 12, 19 December 1950 (1950-12-19), pages 5530-5536, XP055061601, ISSN: 0002-7863, DOI: 10.1021/ja01168a046
- R R Li ET AL: "Gareth Rowlands", , 1 January 2002 (2002-01-01), pages 1-58, XP055344949, Retrieved from the Internet: URL:http://www.massey.ac.nz/~gjrowlan/oxid /meta.pdf [retrieved on 2017-02-13]
- LUCHE JEAN-LOUIS ED - BARNES JONATHAN C ET AL: "Lanthanides in organic chemistry,1. Selective 1,2 reductions of conjugated ketones", JOURNAL OF THE AMERICAN CHEMICAL SOCIETY, AMERICAN CHEMICAL SOCIETY, vol. 100, no. 7, 29 March 1978 (1978-03-29), pages 2226-2227, XP003010366, ISSN: 0002-7863, DOI: 10.1021/JA00475A040

## Description

### Field of the Invention

The present invention relates broadly to a method of preparing certain bile acids and bile acid intermediates. Specifically, it relates to the methods defined in the appended claims. Importantly, the bile acids produced by the method of the invention are not isolated from mammalian and microbial organisms naturally producing these acids and thus are free of any toxins and contaminants associated with such organisms. The present invention relates to the synthesis of deoxycholic acid and pharmaceutically acceptable salts and intermediates thereof.

### Background of the Invention

Cholanology, the study of bile acids, and particularly bile acid chemistry has been of interest for the better part of a century. Although much is known, bile acid chemistry involves a wide variety of chemical entities, many with surprising properties. For a review, *see, e.g.,* Mukhopadhyay, S. and U. Maitra., Current Science 87:1666-1683 (2004) ("Chemistry and biology of bile acids").

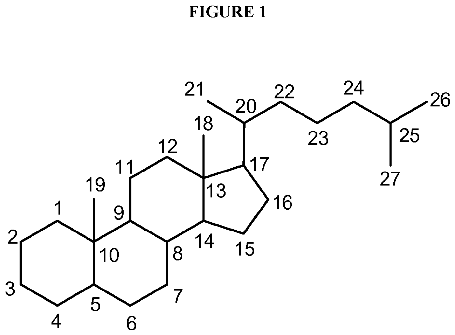

Bile acids are characterized by two connecting units, a rigid steroid nucleus and a short aliphatic side chain (see Figure 1 of the present application). See, Hofmann, A. F., et al. For a proposed nomenclature for bile acids, see J. Lipid Res. 33:599-604 (1992). Both the nucleus and the side chain have a large number of possible steric arrangements. The nucleus can be altered by expansion or contraction of individual rings, and the side chain can be shortened or lengthened. In addition, both parts of the bile acid molecule have a large number of possible polar substituents. Ionizing groups may be present on the nucleus or the side chain. Finally, conjugating groups may be present on the nucleus (e.g., sulfate, glucuronate, phosphate) or on the side chain (glycine or taurine or other amino acids, or even sugars). The side chain structure determines the class of the compound (bile acids or bile salts).

Bile acids are amphiphiles, having both an amphiphilic and amphipathic "face":

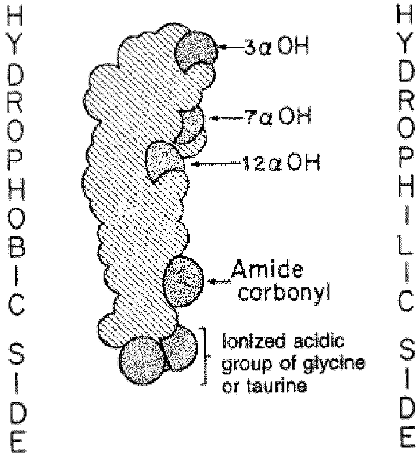

Hofman, A.F., News Physiol. Sci. 14: 24-29 (1999)("Bile Acids: The good, the Bad, and the Ugly", at p. 25 , Figure 1).

By convention, the hydrophobic surface is called the "β-face" and the hydrophilic surface is called the "a-face". The β-face is lipid soluble and the α-face is relatively polar, in general. There are bile acids, such as those having polar groups (hydroxyl groups, in naturally occurring bile acids) on the hydrophobic face as well as on the hydrophilic face, e.g., ursodeoxycholic acid. The amphipathic nature of the molecule is responsible for its forming mixed micelles with amphipathic but waterinsoluble lipids, such as phosphatidylcholine. Bile acids will not solubilize dietary lipids in the form of mixed micelles unless bile acids are above a critical concentration, termed the critical micellization concentration.

The bile acids found in greatest proportion in humans are chenodeoxycholic acid and deoxycholic acid. Deoxycholic acid is also known as deoxycholate, cholanoic acid, and 3α,12α-dihydroxy-5β-cholanate. In the human body deoxycholic acid is used in the emulsification of fats for the absorption in the intestine. In research, deoxycholic acid is used as a mild detergent for the isolation of membrane associated proteins. When substantially pure, deoxycholic acid is a white to off-white crystalline powder form. Deoxycholic acid is one of the four main acids produced by the liver. It is soluble in alcohol and acetic acid. The CAS number for deoxycholic acid is [83-44-3].

Rapid removal of body fat is an age-old ideal, and many substances have been claimed to accomplish such results, although few have shown results. "Mesotherapy", or the use of injectables for the removal of fat, is not widely accepted among medical practitioners due to safety and efficacy concerns, although homeopathic and cosmetic claims have been made since the 1950's. Mesotherapy was originally conceived in Europe as a method of utilizing cutaneous injections containing a mixture of compounds for the treatment of local medical and cosmetic conditions. Although mesotherapy was traditionally employed for pain relief, its cosmetic applications, particularly fat and cellulite removal, have recently received attention in the United States. One such reported treatment for localized fat reduction, which was popularized in Brazil and uses injections of phosphatidylcholine, has been erroneously considered synonymous with mesotherapy. Despite its attraction as a purported "fat-dissolving" injection, the safety and efficacy of these cosmetic treatments remain ambiguous to most patients and physicians. See, Rotunda, A.M. and M. Kolodney, Dermatologic Surgery 32:465-480 (2006) ("Mesotherapy and Phosphatidylcholine Injections: Historical Clarification and Review").

WO 2006/133160 describes methods for lipomodeling, e.g., reduction of a fat depot, by administering a neuropeptide Y receptor antagonist to the site of the fat depot. Kolonin M. G. et al., Nat. Med. June 10(6):625-32 (2004), describes fat selective pro-apoptotic peptides having potent fat cell killing effects. The described pro-apoptotic peptides require access to the vasculature to kill.

Recently published literature reports that deoxycholic acid has fat removing properties when injected into fatty deposits *in vivo. See,* WO 2005/117900 and WO 2005/112942, as well as US2005/0261258; US2005/0267080; US2006/127468; and US20060154906. Deoxycholate injected into fat tissue has two effects: 1) it kills fat cells via a cytolytic mechanism; and 2) it causes skin tightening. Both of these effects are required to mediate the desired aesthetic corrections (i.e., body contouring). Because deoxycholate injected into fat is rapidly inactivated by exposure to protein and then rapidly returns to the intestinal contents, its effects are spatially contained. As a result of this attenuation effect that confers clinical safety, fat removal therapies typically require 4 - 6 sessions. This localized fat removal without the need for surgery is beneficial not only for therapeutic treatment relating to pathological localized fat deposits (e.g., dyslipidemias incident to medical intervention in the treatment of HIV), but also for cosmetic fat removal without the attendant risk inherent in surgery (e.g., liposuction). See, Rotunda et al., Dermatol. Surgery 30: 1001-1008 (2004) ("Detergent effects of sodium deoxycholate are a major feature of an injectable phosphatidylcholine formulation used for localized fat dissolution") and Rotunda et al., J. Am. Acad. Dermatol. 2005: 973-978 ("Lipomas treated with subcutaneous deoxycholate injections").

Pharmaceutical grade bile acid preparations are commercially available at relatively low cost. This low cost is due to the fact that the bile acids are obtained from animal carcasses, particularly large animals such as cows and sheep. Importantly, as with all medicaments from animal sources, there is concern that the animal-derived bile acid products may contain animal pathogens and other harmful agents such as animal or microbial metabolites and toxins, including bacterial toxins such as pyrogens.

Such animal pathogens can include prions, which are thought to be a type of infectious pathogenic protein that may cause prion diseases. Prion diseases are degenerative disorders of the nervous system. One such disease, "Mad cow" disease (thought to be a variant of Creutzfeldt-Jakob disease (CJD)), is thought to be caused by a prion present in edible beef from diseased cows. Most cases are sporadic with unknown mode of transmission; some cases are inherited; and a small number have been transmitted by medical procedures. The spread of human prion diseases through consumption of infected material has been implicated historically in kuru and recently in variant CJD. Other animal prion diseases (scrapie of sheep, transmissible mink encephalopathy, chronic wasting disease of cervids, and bovine spongiform encephalopathy) all seem to be laterally transmitted by contact with infected animals or by consumption of infected feed. Risk assessment and predictions of future events pertaining to prion diseases are difficult to ascertain because of the different modes of transmission, the unpredictable species barriers, the variable distribution of infectivity in tissues, and strain variations found in some diseases.

In general, animal products may be exposed to microbial organisms which produce pyrogens (fever-causing substances). Bacterial contaminants of food and/or pharmaceutical products are also a serious issue as evidenced by contamination of food stuffs by enterohemoragic *E. coli.* Products such as meats derived from cows as well as produce such as apples, spinach, and the like, have been implicated in such contamination. In such cases, it is the toxin produced by the bacteria (rather than the bacteria itself) that produces adverse effects in humans. Such adverse effects include severe diarrhea, kidney failure and in the extreme situations, death. Bacterial endotoxins, a type of pyrogen, must be substantially excluded from all pharmaceutical compositions.

Animal products are generally purified by a process of elimination, i.e., rather than selecting the end-product from a mix, the end product is the material remaining after exclusion of impurities. And, in addition to the potential animal moieties such as pathogens, another artifact of purification from animal sources is that the end-product is a mixture of one or more bile acids. For example, commercial preparations of deoxycholic acid contain some chenodoxycholic acid, as well as cholic acid, which is a precursor to both deoxycholic acid and chenodeoxycholic acid in mammalian bile acid synthesis. Because the exact proportion of deoxy/cheno/cholic is not preselected, this may result in lot-to-lot variation when contemplating manufacturing large amounts of bile acids. Such lot-to-lot variation can be problematic and may engender additional steps in garnering regulatory approvals or quality control, particularly in efforts to produce a pharmaceutical composition. Clearly, producers would desire lot-to-lot predictability in manufacturing bile acid pharmaceutical compositions.

Currently, the concerns regarding animal-derived products containing animal pathogens and other harmful agents has been addressed by sourcing from isolated and inspected animals. For example, deoxycholic acid from animals in New Zealand are a source of bile acids for human use under US regulatory regimes, as long as the animals continue to remain isolated and otherwise free of observable pathogens.

Implicitly, by the need for such governmentally controlled regulatory regime is the recognition of an intrinsic risk of transmission of animal pathogens when animal-derived medicaments are injected. Where non-animal medicament alternatives become available, the governmental regulatory regime is no longer needed. An example of such alternative (non-animal medicament replacing animal-derived medicament) and associated advantages is insulin for human use. The manufacture of beef insulin in the United States was discontinued in 1998, and pork insulin for human use was discontinued in January of 2006. Although animal insulin can be obtained from herds not known to have had exposure to BSE-causing or other pathogenic agents, the manufacturing facilities or processes can expose the animal ingredients to animals which have had exposure to the pathogens. The risk of transmission of pathogenic agents to humans can be eliminated with the use of insulin that is manufactured recombinantly or synthetically. For consumers, the insulin situation is instructive: where synthetic material is freely available, the risk of transmission of animal pathogens is in theory eliminated. For producers, the ability to produce a pure chemical entity that is substantially free of material of animal pathogens is advantageous for safety, quality, and regulatory purposes. Further, a synthetic process typically provides for a more reproducible product than that derived from biological sources.

Presently, because of the relative abundance of animal carcass-derived bile acids, the industry has not taken steps to either fully chemically synthesize bile acids, or prepare bile acids using phytosterol or microbial starting materials. And although bile acid derivatives have been synthesized, this work again primarily involved animal-derived bile acids as starting materials for steroid chemistry, due to the low cost and ready availability of animal materials. Despite historically active efforts in phytosterol research, there are no readily-commercially available phytosterol-derived bile acid pharmaceutical grade compositions. See, e.g., Mukhopadhyay, S. and U. Maitra., Current Science 87: 1666-1683, 1670 (2004) (Noting that the total synthesis of any bile acid had not been performed subject to a 1981 reference, Kametani et al. J. Am. Chem. Soc. 103: 2890 (1981)("First Total Synthesis of (+)-Chenodeoxycholic Acid"). Microbial, such as bacterially-produced bile acids, have been used *in situ* as bacterial products, e.g., for marine oil spill clean-up. See, Maneerat et al., Appl. Microbiol. Biotechnol. 76: 679-683 (2004) ("Bile acids are new products of a mariene bacterium, Myroides sp. Strain SM1"). Ruzena Mickova: "Reduction of 12-oxo derivatives of bile acids", COLLECTION OF CZECHOSLOVAK CHEMICAL COMMUNICATIONS, Vol. 50, 1 January 1985, pages 1239-1243 discloses a reduction process using sodium borohydride in the presence of a cerium salt.

In order to realize the full potential of deoxycholic acid for the removal of fat, it is imperative that the concerns over the use of animal derived products be further addressed. Clearly, there is a need for suitable quantities of efficacious bile acids and related compositions, such as the deoxycholic acids, that are known from the outset to be free from moieties of animal origin (or pathogenic moieties capable of acting in an animal, particularly a mammal, and for human use, having a deleterious effect on a human), and other harmful agents such as animal or microbial metabolites, toxins, including bacterial toxins, such as pyrogens, for use as medicaments in humans. The present invention addresses this concern by providing methods of synthetically preparing bile acid compositions, the resulting bile acids being free of the potential risk of animal pathogens and other harmful agents. The disclosed bile acid compositions can be used in adipolytic therapy and will serve to further advance research and developmental efforts in the area of localized fat removal.

### Summary of the Invention

Adequate quantities of suitable bile acid as a defined pharmaceutical composition is herein provided, as well as methods for synthesis thereof. Bile acid compositions and methods so provided are not isolated from mammalian or microbial organisms that naturally produce the bile acids. In one aspect, particular deoxycholic acid pharmaceutical compositions which are free of all moieties of animal origin and of mammalian and/or bacterial pyrogens, and related methods for production and use are provided. In another aspect, adequate quantities of suitable deoxycholic acids as defined pharmaceutical compositions are provided which can be used as an injectable pharmaceutical composition for localized fat removal, along with related compositions, methods for manufacture and methods of use. The defined deoxycholate injectates of the present disclosure may be combined with a molecule that causes fat to die by an orthogonal mechanism, e.g., NPY antagonists and/or fat selective pro-apoptotic peptides, to provide agents to be used to create a more potent means to mediate body contouring in fewer therapeutic sessions. In another aspect, the present invention provides methods relating to the synthesis of deoxycholic acid and pharmaceutically acceptable salts thereof and intermediates thereto. The synthetically prepared deoxycholic acid can be used in adipolytic therapy for fat removal.

### Brief Description of the Drawings

Figure 1 is a drawing representing the structure of bile acids, including the numbering system for the carbons of the bile acid skeleton.
Figure 2 shows the similarity in dose-dependent decrease in cell survival of primary human adipocytes upon treatment with synthetic sodium deoxycholic acid of the present disclosure in comparison to bovine-derived sodium deoxycholate (Sigma).

### Detailed Description of the Invention

### Definitions

Throughout this disclosure, various publications, patents and published patent specifications are referenced by an identifying citation.

As used herein, certain terms have the following defined meanings. As used in the specification and claims, the singular form "a," "an," and "the" include singular and plural references unless the context clearly dictates otherwise.

Unless otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the following specification and attached claims are approximations. Each numerical parameter should at least be construed in light of the number of reported significant digits and by applying ordinary rounding techniques.

The term "acetylating reagent" refers to a reagent in which can add an acetyl group CH₃C(O)- to a molecule.

The term "acid" refers to a proton donor and includes both organic and inorganic acids.

The term "alkyl" refers to monovalent saturated aliphatic hydrocarbyl groups having from 1 to 10 carbon atoms and preferably 1 to 6 carbon atoms. This term includes, by way of example, linear and branched hydrocarbyl groups such as methyl (CH₃-), ethyl (CH₃CH₂-), *n*-propyl (CH₃CH₂CH₂-), isopropyl ((CH₃)₂CH-), *n*-butyl (CH₃CH₂CH₂CH₂-), isobutyl ((CH₃)₂CHCH₂-), *sec*-butyl ((CH₃)(CH₃CH₂)CH-), *t*-butyl ((CH₃)₃C-), *n*-pentyl (CH₃CH₂CH₂CH₂CH₂-), and neopentyl ((CH₃)₃CCH₂-).

The term "aryl" refers to a monovalent aromatic carbocyclic group of from 6 to 12 carbon atoms having a single ring (*e.g.,* phenyl) or multiple condensed rings (*e.g.,* naphthyl).

The term "animal origin" refers to originating from any of a kingdom (Animalia) of living things including many-celled organisms and single celled organisms.

The term "dehydration reagent" refers to a reagent that can react with water. In one aspect, a dehydration reagent can react with water that is removed from a molecule.

The term "desulfurization reagent" refers to a reagent which can react with a sulfide. In one aspect, a desulfurization reagent can react with a sulfide containing molecule to remove the sulfide group from the molecule.

The term "ethane dithiol or dithiane precursor" refers to a reagent that, with reaction with a carbonyl group, will form an ethane dithiol or dithiane group.

The term "electrophilic acetyl group" refers to an acetyl group as an electrophile, a group which is attracted to electrons and tends to accept electrons.

The term "hydrogenation reagent" refers to a reagent that can donate hydrogen to a molecule.

The term "Lewis acid" refers to an electron pair acceptor. Lewis acids include oraganometallic reagents such as alkyl aluminum halides (e.g. Et₂AlCl and MeAlCl₂).

The term "mammalian origin" refers to originating from any mammalian organism. The term "mammalian organism" refers to a class (Mammalia) of warm-blooded higher vertebrates (as placentals, marsupials, or monotremes) that nourish their young with milk secreted by mammary glands, have the skin usually more or less covered with hair, and include humans.

The term "microbial origin" refers to originating from any microbial organism. The term "microbial organism" refers to a domain (Bacteria) of prokaryotic round, spiral, or rodshaped single-celled microorganisms that may lack cell walls or are gram-positive or gramnegative if they have cell walls, that are often aggregated into colonies or motile by means of flagella, that typically live in soil, water, organic matter, or the bodies of plants and animals, that are usually autotrophic, saprophytic, or parasitic in nutrition, and that are noted for their biochemical effects and pathogenicity.

The term "olefination reagent" refers to regents that react with ketones to form the corresponding olefins. The term "olefin forming conditions" refers to suitable conditions for carryout such transformations. Examples of such reagents include Wittig regeants and Wittig olefination conditions.

The term "oxidizing agent" refers to a reagent which can accept electrons in an oxidation-reduction reaction. In this way, halogen or oxygen can be added to a molecule or hydrogen can be removed from a molecule.

The term "pathogen" refers to a specific causative agent of a disease.

"Pharmaceutically acceptable salt" refers to pharmaceutically acceptable salts derived from a variety of organic and inorganic counter ions well known in the art and include, by way of example only, sodium, potassium, lithium, calcium, magnesium, ammonium, and tetraalkylammonium. Suitable salts include those described in P. Heinrich Stahl, Camille G. Wermuth (Eds.), Handbook of Pharmaceutical Salts Properties, Selection, and Use; 2002. These pharmaceutically acceptable salts can be prepared by reacting DCA with a suitable base. For illustrative purposes, examples of such bases include sodium hydroxide, potassium hydroxide, or lithium hydroxide. Alternatively, the salts can be prepared by hydrolysis of esters of DCA with base and omitting any acidic workup that would lead to DCA.

The term "reducing agent" refers to a reagent which can donate electrons in an oxidation-reduction reaction. In this way, halogen or oxygen can be removed from a molecule or hydrogen can be added to a molecule.

### Compositions and Methods of Use

In various aspects described herein, the present disclosure provides compositions (and useful intermediates) for pharmaceutical use, methods of synthesis thereof, and methods of use of the present pharmaceutical compositions.

Importantly, the present bile acid compositions are free of risks inherent in material obtained from animal starting materials, and therefore do not require the detailed inspections and regulations of animal-derived materials. In one aspect, this disclosure is thus directed to bile acid pharmaceutical compositions free of material of animal origin, such as mammalian pathogens, as well as being substantially free of toxins of bacterial origin, such as pyrogens.

Sodium deoxycholate is a naturally produced bile salt that solubilizes dietary lipids in the digestive tract. It is produced *in vivo* via a complex biosynthetic route utilizing cholesterol as the starting material and involving both human and bacterial enzymes. The primary function of deoxycholate is to assist in the digestive process by solubilizing dietary lipids to facilitate absorption. In the body, deoxycholate biosynthesis begins with the enzymatic oxidation, isomerization, and reduction of cholesterol in the liver to form cholic acid, a bile acid structurally similar to its cholesterol parent (Stryer L, Chapter 27: Biosynthesis of Membrane Lipids and Steroids, in Biochemistry, 1995, W. H. Freeman and Company: New York. p. 691-707). In the liver, cholic acid is then chemically linked to one of two amino acids (taurine or glycine) to form the 'conjugated' cholic acids (*i.e.,* L-glycocholate and taurocholate). These conjugated cholic acids are then stored in the gall bladder until food consumption. After food consumption, bile solution is released from the gall bladder into the intestine, where the conjugated cholic acid molecules are subject to two additional chemical modifications mediated by enzymes produced by intestinal microflora (Ridlon J.M., Kang D.J. and Hylemon P.B., Bile salt biotransformations by human intestinal bacteria, J. Lipid Res., 47(2): p. 241-59 (2006)). First, conjugated cholic acid is dehydroxylated to form conjugated deoxycholate. Conjugated deoxycholate is then deconjugated to form free deoxycholate, which participates, along with the other bile acids, in the solubilization of dietary lipids. Because deoxycholate is downstream from cholic acid synthesis, cholic acid may be an impurity present in natural sources of deoxycholate.

Deoxycholate is soluble to 333 mg/mL in water, sparingly soluble in alcohol, and is even less soluble in acetone and glacial acetic acid. Reversible formation of micelles may occur with sodium deoxycholate concentrations above the critical micelle concentrations of approximately 2.4 mg/mL and neutral pH (Matsuoka K, M.Y., Micelle formation of sodium deoxycholate and sodium ursodeoxycholate (part 1), Biochim. Biophys. Acta., 1580(2-3): p. 189-99 (2002)). At concentrations above the critical micelle concentration of 2.4 mg/mL, deoxycholate will form micelles and has the ability to solubilize cells, lipids, and proteins. At lower concentrations such as 0.4 mg/mL (comparable to the fasting state), deoxycholate is 98% bound to albumin (Roda A. et al., Quantitative aspects of the interaction of bile acids with human serum albumin, J. Lipid Res., 23(3): p. 490-5 (1982)) in the presence of 26 mg/mL of albumin (which is close to the serum physiological concentration of 35-50 mg/mL).

The preferred embodiments are directed to deoxycholic acid (DCA) or a prodrug thereof or a pharmaceutically acceptable salt of the compound or the prodrug and the related compositions and methods, wherein deoxycholic acid (DCA) is: 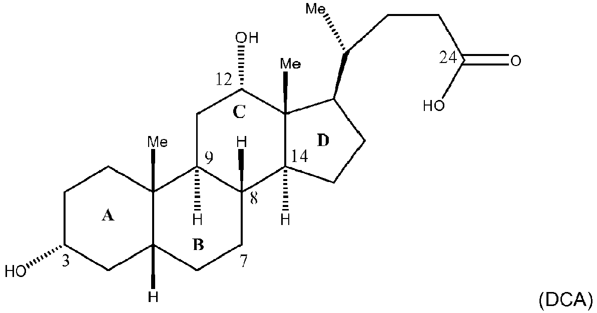 wherein said compound is not isolated from a mammalian or microbial organism naturally producing DCA.

Other preferred embodiments also are directed to stereoisomers of DCA and pharmaceutically acceptable salts thereof and to intermediates in the synthesis of the DCA and its stereoisomers and salts and the related compositions and methods.

The present bile acid pharmaceutical compositions are optionally in salt form, and, further optionally contain a pharmaceutically acceptable diluent, excipient or carrier. In one aspect, this disclosure is directed to a compound that is deoxycholic acid (DCA) or a prodrug thereof or a pharmaceutically acceptable salt of the compound or the prodrug: 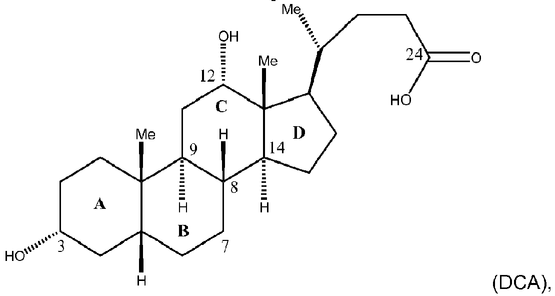 wherein said compound is not isolated from a mammalian or microbial organism naturally producing DCA and a pharmaceutically acceptable excipient.

Preferred cations for salt preparation may be selected from the group consisting of sodium (Na⁺), potassium (K⁺), lithium (Li⁺), magnesium (Mg²⁺), calcium (Ca²⁺), barium (Ba²⁺), strontium (Sr²⁺), and ammonium (NH₄⁺). Salts may also be prepared from an alkali metal or an alkaline earth metal. An alkali metal may be selected from among sodium (Na⁺), potassium (K⁺), and lithium (Li⁺). An alkaline earth metal may be selected from the group consisting of magnesium (Mg²⁺), calcium (Ca²⁺), barium (Ba²⁺), and strontium (Sr²⁺). Preferably for use as a pharmaceutical composition for localized removal of fat, the bile salt is sodium deoxycholate.

Prodrugs of the compounds of the embodiments are also contemplated. A prodrug is an active or inactive compound that is modified chemically through *in vivo* physiological action, such as hydrolysis, metabolism and the like, into a compound of the embodiments following administration of the prodrug to a patient. For example, one may prepare a C₁-C₁₀ ester or an amide of the present deoxycholic acid or derivatives thereof, so that the release of the deoxycholic acid or derivatives thereof is triggered by the disruption of the cell membrane, and release of esterase. With the release of esterase, the ester protecting group is cleaved so that the deoxycholic acid active form or derivatives thereof is present at the desired location *in situ.* Such C₁-C₁₀ esters can optionally include 1-4 heteroatoms selected from oxygen, sulfur or nitrogen; alkyl groups such as methyl, ethyl, isopropyl, butyl, hexyl etc. optionally having 1-4 heteroatoms selected from oxygen, sulfur or nitrogen; alkylphenyl groups having a total of up to 10 carbon atoms, such as, a benzyl or an ethyl phenyl group optionally having 1-4 heteroatoms at any acceptable point of substitution; and an aryl group such as a phenyl group. Example of amide includes, but is not limited to, hydroxamate. For a general discussion of prodrugs involving esters see Svensson and Tunek Drug Metabolism Reviews 165 (1988) and Bundgaard Design of Prodrugs, Elsevier (1985). Synthesis of a C₁-C₁₀ ester or an amide of the deoxycholic acid is well known in the art. For example, an ester can be synthesized by a reaction of the deoxycholic acid with an alcohol in the presence of a mineral acid, in an esterification reaction.

Native structures of deoxycholic acid and chenodeoxycholic acid are shown as below. The four rings (A, B, C, D) are shown as well as the carboxylic acid side chain that extends from the D-ring.

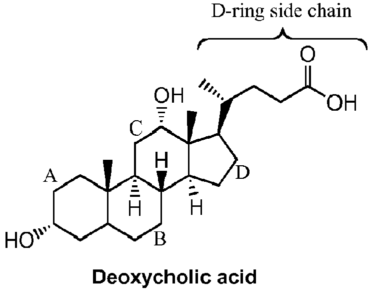

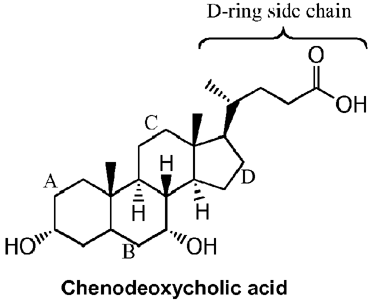

Without limitation, some of the examples of esters, hydroxamates, and hydroxyamides of the deoxycholic acid as well as chenodeoxycholic acid (CDCA) or derivatives thereof, are described as below. Different functional groups can be attached to deoxycholic acid or chenodeoxycholic acid by esterification of the carboxylic group on the D-ring side chain to generate prodrugs. Without limitation, some examples of these D-ring side chain esters are shown in Table 1.

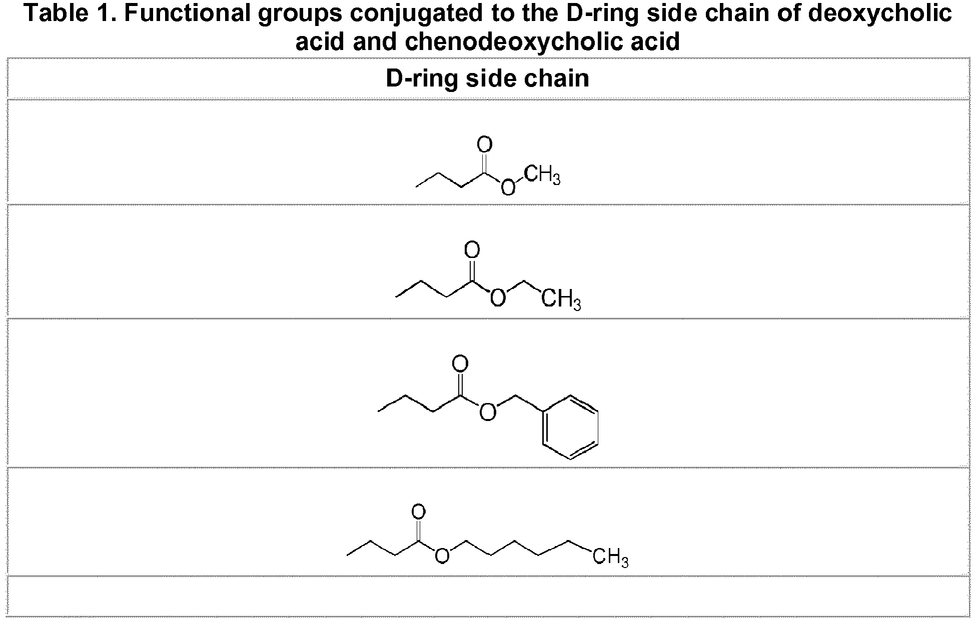

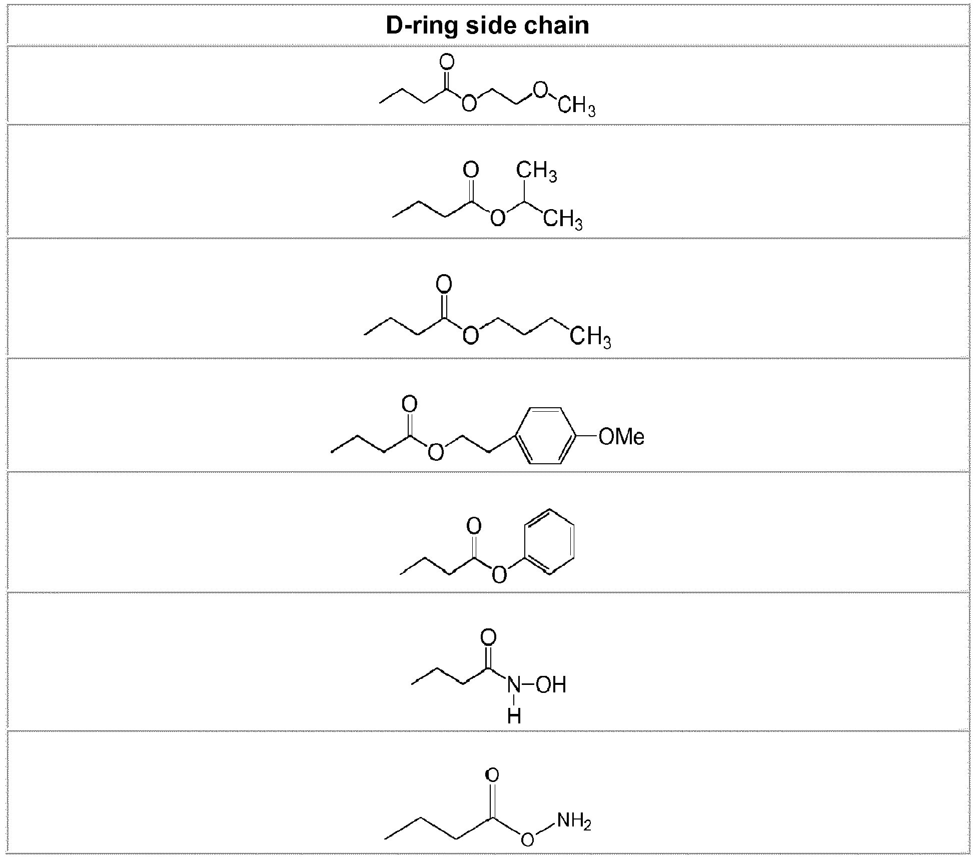

Release of the deoxycholic acid or chenodeoxycholic acid and derivates thereof can be triggered by disruption of the cell membrane and the release of esterase. With the release of esterase, the ester protecting group can be cleaved so that the deoxycholic acid's or chenodeoxycholic acid's active form or derivative thereof is present at the desired location *in situ.*

Prodrugs of deoxycholic acid, chenodeoxycholic acid and their derivatives also include epimers that may possess opposite stereochemistry from the native molecule. Examples of these epimeric molecules are shown in Table 2.

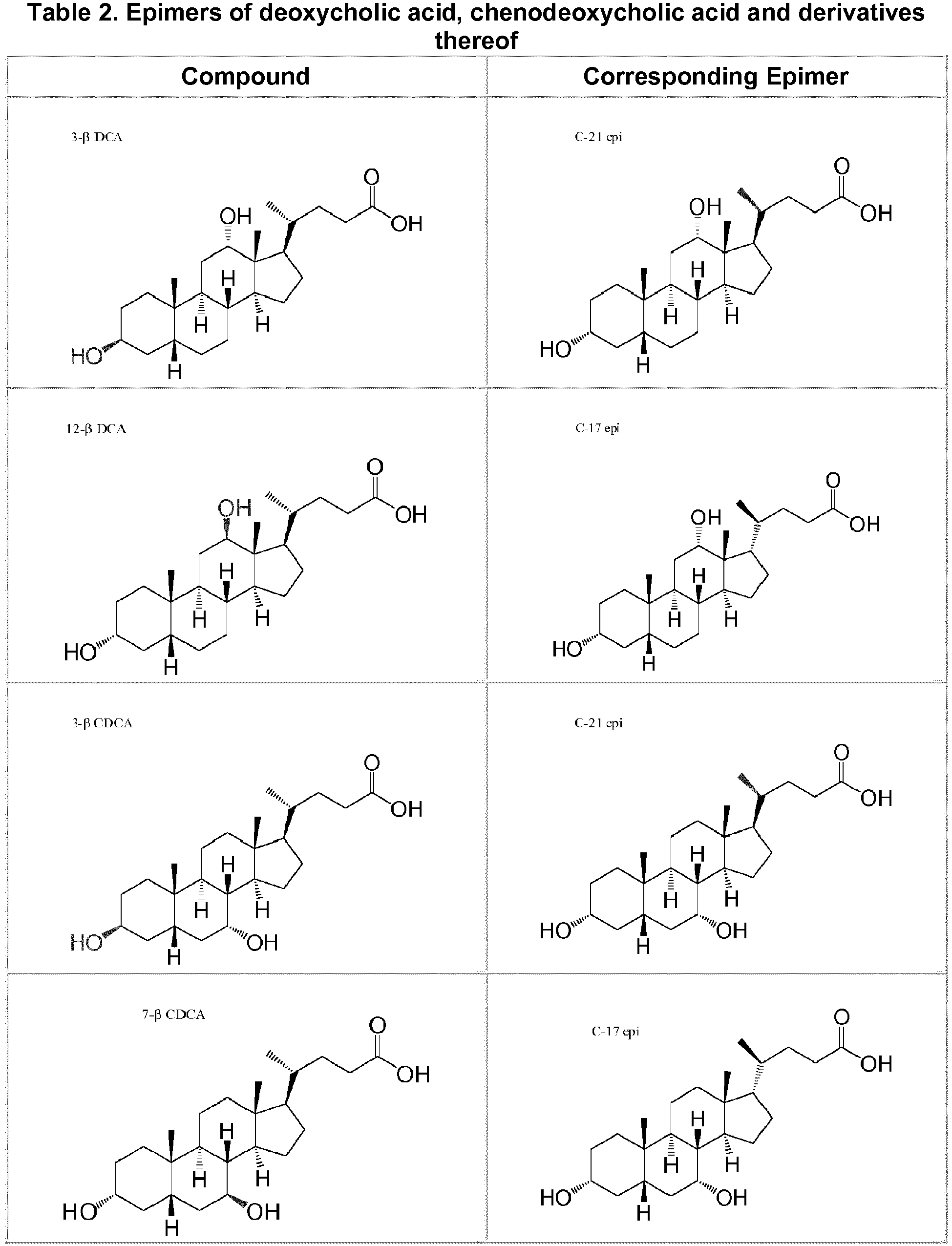

There may be a local irritation upon injection of a bile acid composition of the present embodiments, and thus it may be desirable to administer, simultaneously or *in seriatim,* a local anesthetic. For example, lidocaine is frequently used in humans, and may be administered either as a co-formulation (in the same container and injected at the same time) or co-injection (injected from a different container). Anesthetics such as lidocaine may be administered via topical preparation, such as a patch or ointment.

For deeper tissue, anesthetics may be more deeply injected into the subject tissue or administered systemically (e.g., general anesthesia, epidural or other known methods).

The bile acid(s) or bile salt(s) in a solution of the disclosure can be at a concentration of about 0.001 to 10, 0.01 to 5, or 0.1 to 2% w/w, w/v, or v/v. Preferably, the bile acid(s) or bile salt(s) in the above solution can be at a concentration of about 0.1-5 % w/w or more preferably about 1% w/w. In some embodiments, the fat dissolving solution comprises up to 100, 50, 20, 10, 5, 2, 1, 0.5, 0.2, 0.05, 0.02, or 0.01 grams of the one or more detergents, bile acids and/or bile salts, e.g., deoxycholic acid or salts thereof or sodium deoxycholate.

In preferred embodiments, the solutions herein include no lipids, phospholipids, or phosphatidylcholine. In some embodiments, the solutions herein include up to 5% w/w, w/v, or v/v lipids, phospholipids, or phosphatidylcholine.

In some embodiments, the above solution can further comprise a second therapeutic agent selected from the group consisting of: anti-microbial agents, vasoconstrictors, anti-thrombotic agents, anti-coagulation agents, suds-depressants, anti-inflammatory agents, analgesics, dispersion agents, anti-dispersion agents, penetration enhancers, steroids, tranquilizers, muscle relaxants, and anti-diarrhea agents. In some embodiments, a solution is in a container that contains up to 500 mL of solution. Such container can be a syringe or syringe-loadable container.

In some embodiments, compositions and methods further comprise a molecule known to cause fat to die by an orthogonal mechanism. Such molecules include neuropeptide Y (NPY) receptor antagonists including, but not limited to, NPY receptor antagonists, such as BIBP-3226 (Amgen), BIBO-3304 (Boehringer Ingleheim), BMS-192548 and AR-H040922 (Bristol-Myers Squibb), LY-357897 (Eli Lilly), 1229U91 and GW438014S (GlaxoSmithKline), JNJ-5207787 (Johnson & Johnson), Lu-AA-44608 (Lundbeck), MK-0557 (Merck NPY), NGD-95-1 (Neurgogen), NLX-E201 (Neurologix), CGP-71683 (Novartis), PD-160170 (Pfizer), SR-120819A, BIIE0246, and S.A.0204 (Sanofi Aventis), S-2367 (Shiongli), dihydropyridine and dihydropyridine derivatives that are NPY receptor antagonists, bicyclic compounds that are NPY receptor antagonists, carbazole NPY receptor antagonists, and tricyclic compounds that are NPY receptor antagonists. *See, e.g.,* WO 2006/133160 and U.S. 6,313,128. Also contemplated are fat selective pro-apoptotic peptides such as the CKGGRAKDC peptide that homes to white fat vasculature. See, Kolonin M.G. et al., Nat. Med. June 10(6):625-32 (2004).

In one aspect, the present disclosure relates to methods for reducing a subcutaneous fat deposit in a subject. Such methods comprise the step of administering locally to a subcutaneous fat deposit in the subject a composition comprising: (i) a fat-dissolving effective amount of one or more pharmacologically active detergents, or bile acid(s) and/or bile salt(s), or deoxycholic acid or a salt thereof, or sodium deoxycholate; (ii) a pharmaceutical, veterinary, or cosmetic excipient; and (iii) optionally a lipid, wherein the ratio of the lipid and bile acid or bile salt is up to 1% w/w and wherein the composition does not include lipase or colipase. In some embodiments, the fat deposit is associated with a condition selected from the group consisting of obesity, fat redistribution syndrome, eyelid fat herniation, lipomas, Dercum's disease, lipodystrophy, buffalo hump lipodystrophy, dorsocervical fat, visceral adiposity, breast enlargement, hyperadiposity, diffused body fat around trunk and arms, and fat deposits associated with cellulite. In preferred embodiments, the above method does not include performing surgery on said subject.

In one aspect, this disclosure is directed to a method for removal of fat deposits from selected locations in a mammal comprising administering to the mammal in need thereof a therapeutically effective amount of DCA or a prodrug thereof or a pharmaceutically acceptable salt of the DCA or the prodrug: 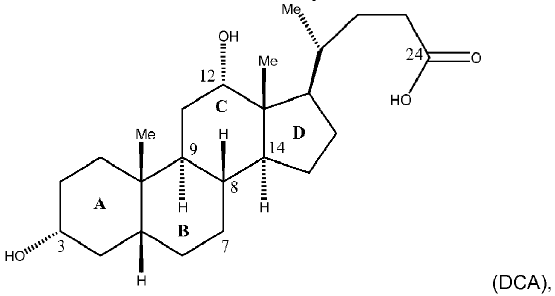 wherein said compound is not isolated from a mammalian or microbial organism naturally producing DCA.

In one embodiment, the methods of this disclosure may further comprise administering to the mammal at least one additional active ingredient selected from the group consisting of a neuropeptide Y (NPY) receptor antagonist and a fat selective pro-apoptotic peptide. In one embodiment, the neuropeptide Y (NPY) receptor antagonist is selected from the group consisting of BIBP-3226, neuropeptide Y5 antagonist (the Amgen NPY receptor antagonists), BIBO-3304 (the Boehringer Ingleheim NPY receptor antagonist), BMS-192548 (the Bristol-Myers Squibb NPY receptor antagonist), AR-H040922 (the Bristol-Myers Squibb NPY receptor antagonist), LY-357897 (the Eli Lilly NPY receptor antagonist), the Esteve NPY-Y5 receptor antagonist, 1229U91 (the GlaxoSmithKline NPY receptor antagonists), GW438014S (the GlaxoSmithKline NPY receptor antagonists), JNJ-5207787 (the Johnson & Johnson NPY receptor antagonist), Lu-AA-44608 (the Lundbeck NPY receptor antagonist), MK-0557 (the Merck NPY receptor antagonist), NGD-95-1 (the Neurgogen NPY receptor antagonist), NLX-E201 (the Neurologix NPY receptor antagonist), CGP-71683 (the Novartis NPY receptor antagonist), PD-160170 (the Pfizer NPY receptor antagonists), SR-120819A (the Sanofi Aventis NPY receptor antagonists), BIIE0246 (the Sanofi Aventis NPY receptor antagonists), S.A.0204 (the Sanofi Aventis NPY receptor antagonists), S-2367 (the Shiongli NPY receptor antagonist), dihydropyridine that are NPY receptor antagonists, dihydropyridine derivatives that are NPY receptor antagonists, bicyclic compounds that are NPY receptor antagonists, carbazole NPY receptor antagonist, and tricyclic compounds that are NPY receptor antagonists. *See, e.g.,* WO 2006/133160 and U.S. 6,313,128. In one embodiment, the fat selective pro-apoptotic peptide is CKGGRAKDC peptide that homes to white fat vasculature. See, Kolonin M.G. et al., Nat. Med. June 10(6):625-32 (2004).

In one aspect, the present disclosure relates to methods for reducing the appearance of a skin condition in a skin region of a subject. Such methods comprise the step of: administering locally to said skin region a composition comprising: (i) a skintightening effective amount of one or more pharmacologically active detergents, or bile acid(s) and/or bile salt(s), or deoxycholic acid or a salt thereof, or sodium deoxycholate, (ii) a pharmaceutical, veterinary, or cosmetic excipient, and (iii) optionally a lipid. In some embodiments, the administering step involves delivering the compositions herein via a subcutaneous or transdermal injection. In some embodiments, the skin condition being treated or ameliorated is selected from the group consisting of: loose skin, skin aging, irregularities of the skin, and wrinkles. In some embodiments, the region of skin being treated is under eye, under chin, under arm, buttock, cheek, brow, calf, back, thigh, ankle, or stomach.

In some embodiments, the compositions used for reducing the appearance of a skin condition in a skin region are formulation into a skin tightening solution. Such skin tightening solution can further comprise a second therapeutic agent selected from the group consisting of: anti-microbial agents, vasoconstrictors, anti-thrombotic agents, anti-coagulation agents, suds-depressants, anti-inflammatory agents, analgesics, dispersion agents, anti-dispersion agents, penetration enhancers, steroids, tranquilizers, muscle relaxants, and anti-diarrhea agents.

In preferred embodiments, the detergent comprises a bile acid selected from the group consisting of deoxycholic acid, cholic acid, chenodeoxycholic acid, 7-alpha-dehydroxylate chenodeoxycholic acid, lithocholic acid, ursodeoxycholic acid, dihydroxytaurin acid, trihydroxytaurine acid, and glycine conjugates of any of the above. In some embodiments, the detergent comprises a bile salt that includes a cation selected from the group consisting of sodium (Na⁺), potassium (K⁺), lithium (Li⁺), magnesium (Mg²⁺), calcium (Ca²⁺), barium (Ba²⁺), strontium (Sr²⁺), and ammonium (NH₄⁺). In some embodiments, the detergent comprises a bile salt with a cation that is an alkali metal or an alkaline earth metal. Preferably, the alkali metal is sodium (Na⁺), potassium (K⁺), or lithium (Li⁺) and the alkaline earth metal is magnesium (Mg²⁺), calcium (Ca²⁺), barium (Ba²⁺), or strontium (Sr²⁺). More preferably, the bile salt is sodium deoxycholate.

Another embodiment provides for a method of emulsifying fat in a mammal comprising administering to the mammal in need thereof a therapeutically effective amount of a compound that is DCA or a prodrug thereof or a pharmaceutically acceptable salt of the DCA or the prodrug: 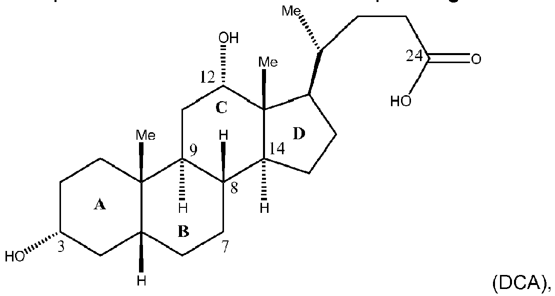 wherein said compound is not isolated from a mammalian or microbial organism naturally producing DCA.

Another embodiment provides for a method of solubilizing phosphatidylcholine comprising mixing phosphatidylcholine and effective amount of a compound that is DCA or a prodrug thereof or a pharmaceutically acceptable salt of the DCA or the prodrug, 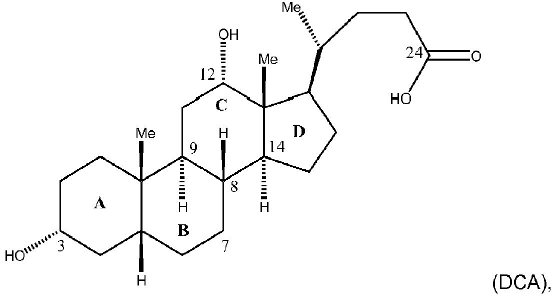 wherein said compound is not isolated from a mammalian or microbial organism naturally producing DCA.

Another aspect of the disclosure relates to mixing adipo-ablative bile acids, such as, deoxycholic acid (DCA) with agents that kill fat cells. In one aspect, this disclosure contemplates a means to enhance the aesthetic effects of deoxycholate injections by mixing into the deoxycholate injectate a molecule that causes fat to die by an orthogonal mechanism. Examples of such candidate molecules include, but are not limited to, neuropeptide Y (NPY) antagonists and fat selective pro-apoptotic peptides, as described herein. Since both fat cell killing and skin tightening may be required to mediate the desired effects, the effects of an agent with fat killing ability and potent skin tightening effects (such as deoxycholate) can be enhanced via the addition of a molecule with potent fat cell killing effects. Additionally, molecules that require access to the vasculature to kill (such as certain pro-apoptotic peptides that bind to proteins expressed on the luminal side of capillaries) can gain access to these proteins because deoxycholate may cause vascular leakage. Thus, such agents can be synergistic with deoxycholate potentially creating a more potent means to mediate body contouring in fewer therapeutic sessions.

### Method of Synthesis

The present invention provides methods of synthesis of the compounds, salts, and prodrugs of DCA and their related intermediates.

The numbering of the steroidal scaffold as used herein follows the general convention as shown in Figure 1.

Accordingly, provided is a method for preparing deoxycholic acid (DCA) or an ester thereof or a pharmaceutically acceptable salt thereof: 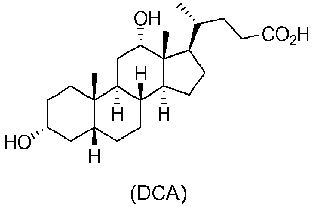 the method comprising
1. (a) reacting 9α-hydroxyandrost-4-en-3,17-dione **1** with H₂ under hydrogenation conditions to form compound **2** 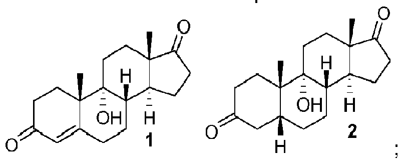
2. (b) reacting compound **2** with acid to form compound **3** 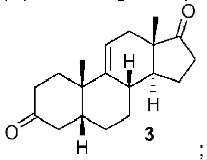
3. (c) reacting compound **3** with a reducing agent to form compound **4** as a mixture of **4** and **5** 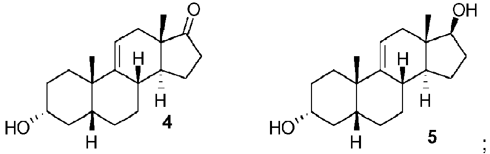
4. (d) reacting compound 4 with a two carbon olefination reagent under olefin forming conditions to form compound **6** 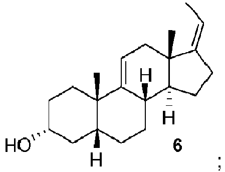
5. (e) converting compound **6** to a compound of formula **7** wherein P is a protecting group 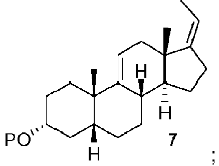
6. (f) reacting a compound of formula **7** with an alkylpropiolate CH≡C-C(O)OR or an alkyl acrylate CH₂=CHC(O)OR wherein R is alkyl in the presence of a Lewis acid to form a compound of formula **8** wherein P is a protecting group, R is alkyl, and the dashed line ---- is a single or double bond; 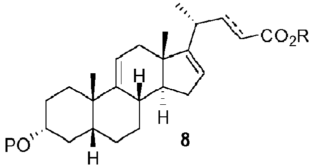
7. (g) reacting a compound of formula **8** with H₂ under hydrogenation conditions to form a compound of formula **9** wherein P is a protecting group and R is alkyl 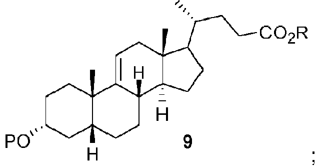
8. (h) reacting compound of formula **9** with an oxidizing agent to form a compound of formula **10** wherein P is a protecting group and R is alkyl 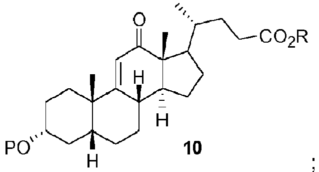
9. (i) reacting a compound of formula **10** with H₂ under hydrogenation conditions to form compound of formula 11 wherein P is a protecting group and R is alkyl 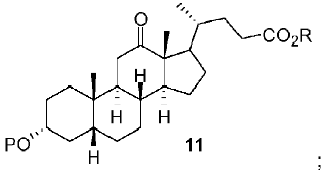
10. (j) reacting compound of formula **11** with a reducing agent to form a compound of formula **12** wherein P is a protecting group and R is alkyl 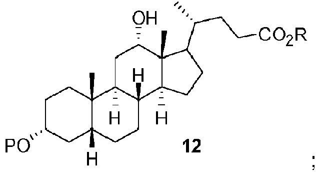 and
11.(k) exposing compound of formula **12** to deprotection conditions to form an ester thereof and optionally to suitable hydrolysis conditions to form deoxycholic acid or the pharmaceutically acceptable salt thereof.

The present disclosure also provides the following intermediates shown in Scheme 1 below wherein P and R are as defined above.

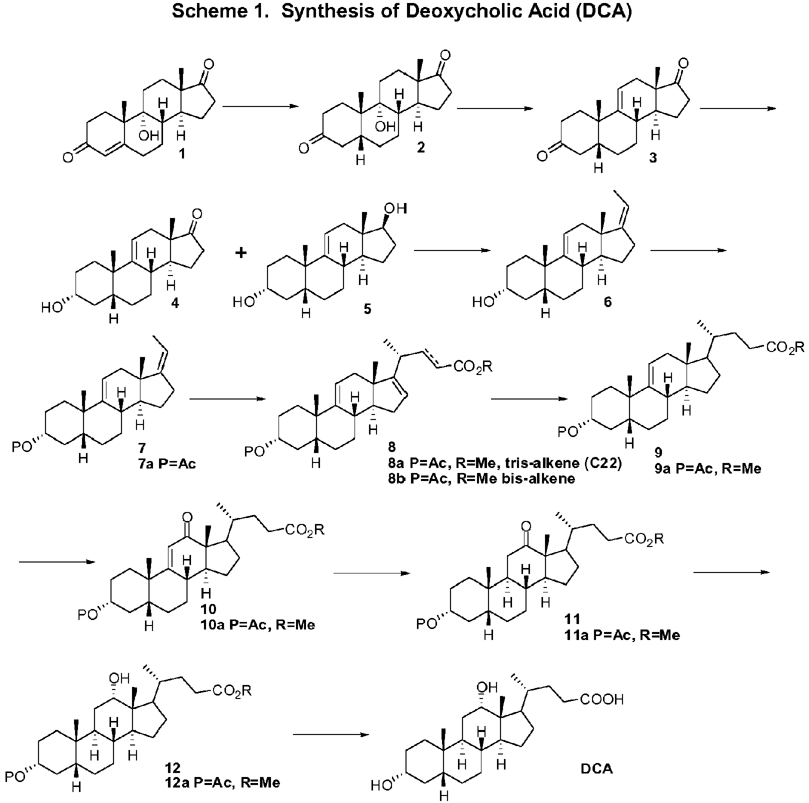

In one embodiment, the hydrogenation conditions of part (a) comprises a Pd/C catalyst.

In one embodiment, the acid of part (b) is a mineral acid. In some aspects, the mineral acid is H₂SO₄.

In one embodiment, the reducing agent of part (c) is LiAl(OtBu)₃H.

In one embodiment, the two carbon olefination reagent of part (d) is a Wittig agent such as Ph₃PCH₂CH₃⁺Br⁻.

In one embodiment, the protecting group P of compound 7-12 is -C(O)CH₃. In some aspects, compound 6 is exposed to acylation conditions to form 7a, such as by treatment of 6 with acetic anhydride and an organic base such as Et₃N, pyridine, and/or dimethylaminopyridine.

In one embodiment, the Lewis acid of part (f) is EtAlCl₂.

In one embodiment, the alkylpropiolate of part (f) is methylpropriolate.

In one embodiment, the alkyl acrylate of part (f) is methylacrylate.

In one embodiment, the hydrogenation conditions of part (g) comprises a PtO₂ or Pd/C catalyst.

In one embodiment, the oxidizing agent of part (h) is CrO₃.

In one embodiment, the hydrogenation conditions of part (i) comprises a Pd/C catalyst.

In one embodiment, the reducing agent of part (j) is LiAl(OtBu)₃H.

In one embodiment, the deprotection and hydrolysis conditions of part (k) when P is -C(O)CH₃ comprises reacting compound 12 with an alkali earth hydroxide, alkali earth alkoxide, or a mixture of both. In some aspects, the hydrolysis conditions include an acidic workup to give deoxycholic acid. In other aspects, the acidic workup is omitted to give the corresponding salt.

In one embodiment, the alkali metal hydroxide is LiOH.

In one embodiment, salts of deoxycholic acid can be prepared by reaction with an alkali earth metal alkoxide or hydroxide. Salts of deoxycholic acid include the sodium (Na⁺), potassium (K⁺), and lithium (Li+) salts.

In one embodiment, provided is an intermediate compound selected from the group consisting of
9α-Hydroxy-5β-androstan-3,17-dione **(2);**
5β-Androst-9(11)-en-3,17-dione **(3);**
(Z)-3α-Hydroxy-5β-pregna-9(11),17(20)-diene **(6);**
(Z)-3α-Acetoxy-5β-pregna-9(11),17(20)-diene **(7a);**
(E)-Methyl 3α-acetoxy-5β-chol-9(11), 16, 22-trien-24-oate **(8a);**
Methyl 3α-acetoxy-5β-chol-9(11), 16-dien-24-oate **(8b);**
Methyl 3α-acetoxy-5β-chol-9(11)-en-12-one-24-oate **(10a);** and
Methyl 3α-acetoxy-5β-cholan-12-one-24-oate **(11 a).**

### Pharmaceutical compositions and modes of administration

The compositions can be comprised of a disclosed compound in combination with at least one pharmaceutically acceptable excipient. Acceptable excipients are non-toxic, aid administration, and do not adversely affect the therapeutic benefit of the disclosed compound. Such excipient may be any solid, liquid, semi-solid or, in the case of an aerosol composition, gaseous excipient that is generally available to one of skill in the art.

Solid pharmaceutical excipients include starch, cellulose, talc, glucose, lactose, sucrose, gelatin, malt, rice, flour, chalk, silica gel, magnesium stearate, sodium stearate, glycerol monostearate, sodium chloride, dried skim milk and the like. Liquid and semisolid excipients may be selected from glycerol, propylene glycol, water, ethanol and various oils, including those of petroleum, vegetable or synthetic origin, e.g., peanut oil, soybean oil, mineral oil, sesame oil, etc. Preferred liquid carriers, particularly for injectable solutions, include water, saline, aqueous dextrose, and glycols.

In general, the compounds of preferred embodiments will be administered in a therapeutically effective amount by any of the accepted modes of administration for agents that serve similar utilities. The actual amount of the compound of preferred embodiments, i.e., the active ingredient, will depend upon numerous factors such as the severity of the disease to be treated, the age and relative health of the subject, the potency of the compound used, the route and form of administration, and other factors. The drug can be administered more than once a day, preferably once or twice a day. All of these factors are within the skill of the attending clinician.

The amount of the compound in a formulation can vary within the full range employed by those skilled in the art. The present compositions in various aspects as described herein may be prepared wherein the deoxycholic acid moiety is in the range of about 0.5%-10% on a weight per aqueous volume basis, or, on a w/w basis assuming the density of water (i.e., a 1:1 correspondence between weight and volume). In another aspect, the present embodiments relate to the presently described pharmaceutical compositions in concentrations up to saturation point of the diluent. One may select the degree of thixotropic viscosity based on conditions such as concentration and pH. *See, e.g.,* Mukhopadhyay, S. and U. Maitra, Current Science 87: 1666-1683 (2004) at 1680.

In some embodiments, the administering step involves delivering the compositions herein via a dermal patch, a pump, or subdermal depot. In some embodiments, the administering step involves delivering the compositions herein topically or subcutaneously. In specific embodiments, the administration step involves administering locally (e.g., subcutaneously or subdermally) to a region under eye, under chin, under arm, buttock, calf, back, thigh, or stomach of said subject. The administration can be made by a subcutaneous or transdermal injection.

### Synthetic schemes

Other examples of methods for complete chemical synthesis of bile acid pharmaceutical compositions, and useful intermediates, are provided below.

These following descriptions and examples provide an alternative to the extraction of DCA from mammalian or microbial organisms that naturally produce this compound. Synthetic routes 1-6 are contemplated for use in the present invention to synthesize deoxycholic acid (DCA). Synthetic route 1B and Examples 1-11 show the synthesis of DCA from hydrocortisone.

### 1. Synthetic route #1A from Adrenosterone, via 9(11)-ene or 11,12-ene

Cortisone (Compound 1.1) of Scheme 1A (below) is widely available as a fully synthetic material. It can be efficiently cleaved to form the C₁₇ ketone compound using pyridinium chlorochromate (PCC). This cleavage to adrenosterone (Compound 1.2) can also be achieved using HIO₄ or sodium bismuthate (NaBiO₃). The reaction that converts Compound 1.2 to Compound 1.3 is a known chemical process. Conversion of Compound 1.3 into Compound 1.4 involves monoketalization. Subsequent steps are regeneration of the 3-keto-4-ene, selective reduction of the 4,5-ene (H₂/Pt/DMF) to yield the C₅β-configuration and selective reduction of the C₃ carbonyl group to the desired 3α-configuration to yield compound 1.5. The addition of a protecting group in converting Compound 1.5 into Compound 1.6 and subsequent reduction of the product yields the C₁₁ β-ol (axial configuration), i.e., compound 1.7, which is suitable for regioselective elimination to the key 9(11)-ene (i.e., conversion of Compound 1.7 into Compound 1.8).

The synthetic scheme bifurcates here, in that Compound 1.7 can be used as the starting material for conversion to either Compound 1.8 or Compound 1.9. The elimination reaction used to convert Compound 1.7 into Compound 1.8 is regioselective because of the trans diaxial relationship between the C₁₁ hydroxyl group and C₉ hydrogen atom. The alternative mode of elimination to yield the isomeric C₁₁-C₁₂ olefin of compound 1.9 is likewise regioselective involving *cis-*thermal elimination (i.e., conversion of Compound 1.7 to Compound 1.9).

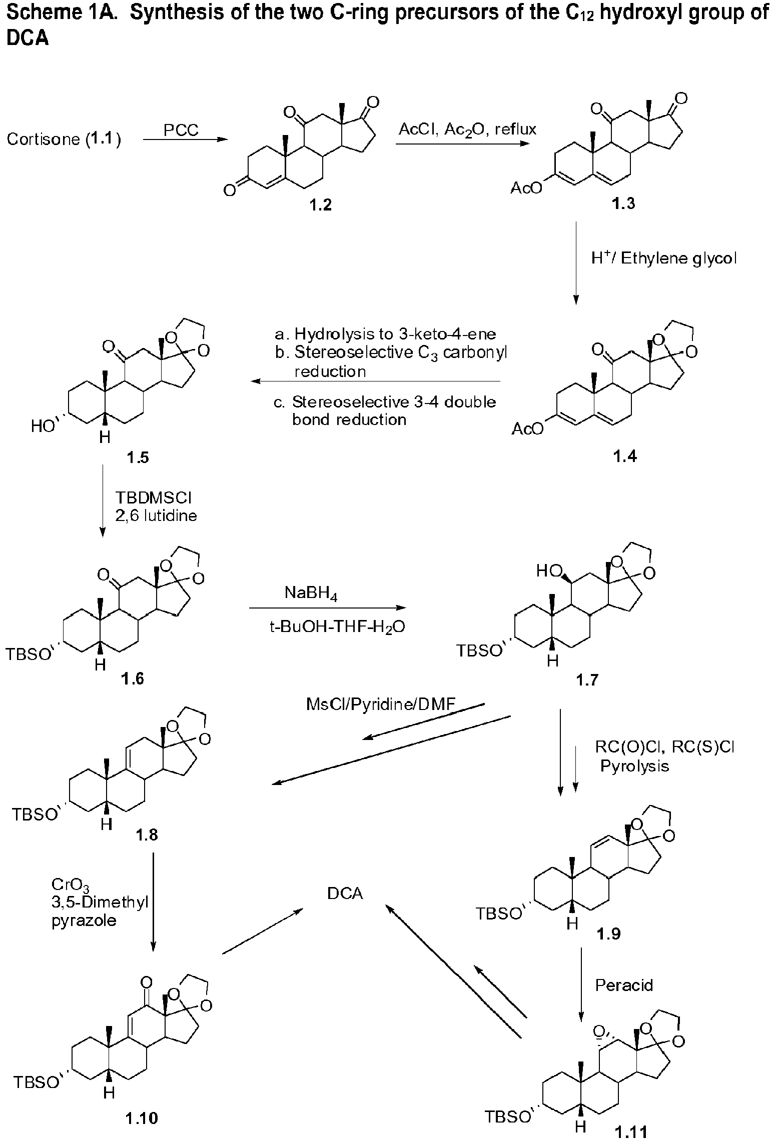

Allylic oxidation of Compound 1.8(via treatment with CrO₃ and 3,5 dimethyl pyrazole) yields the enone-containing Compound 1.10. Peracid oxidation of Compound 1.9 proceeds stereoselectively from the alpha-face of the steroid to yield the C₁₁₋₁₂ epoxide Compound 1.11 (see Scheme 1A above). These chemical transformations yield the two key precursors of the C₁₂ hydroxyl group functionality, namely, Compound 1.10 and Compound 2.1 (Schemes 1A and 2).

One of the skill in the art will appreciate that the above cortisone route can be modified to begin instead with hydrocortisone, which has the same carbon skeleton and the same relative placement of oxygen atoms, with hydrocortisone differing from cortisone only in the oxidation state of the C-11 oxygen bearing carbon atom. Hydrocortisone is commercially available and various synthesis of this compound are known (Szczebara et. al. Nature Biotechnology 21:143-149 (Feb. 2003)) including a total chemical synthesis (Woodward R. B. et. al. J. Am. Chem. Soc. 74: 4223 (1952)). Ketone 1.13 is synthesized starting from hydrocortisone 1.12 (Scheme 1 B) via hydrogenolysis of the α,β-unsaturated double bond, followed by global ketone reduction using sodium borohydride to allow for 1,2-diol cleavage using NalO₄, thus forming the C₁₇ ketone on the D-ring of the steroidal ring system. Subsequent oxidation with pyridinium chlorochromate (PCC) yields 1.13. Treatment of 1.13 with K-selectride® followed by acetylation with acetic anhydride/pyridine gives protected alcohol 1.15. Subsequent olefination of 1.15 with a Wittig reagent provides alkene 1.16 that is then treated with methyl propiolate and ethyl aluminum dichloride to form diene 1.17. Following hydrogenation of both double bonds, ketone 1.18 is reduced and the resulting alcohol intermediate is eliminated upon treatment with SOCl₂ in pyridine to give alkene 1.19. Allylic oxidation of alkene 1.19 with CrO₃ and reduction of the double bond under hydrogenation conditions gives ketone 1.21. Removal of the acetate protecting group and oxidation of the resulting alcohol gives diketone 1.22. Reduction of 1.22 with LiAlH(O-*^{t}*Bu)₃ and hydrolysis of the methyl ester yields DCA.

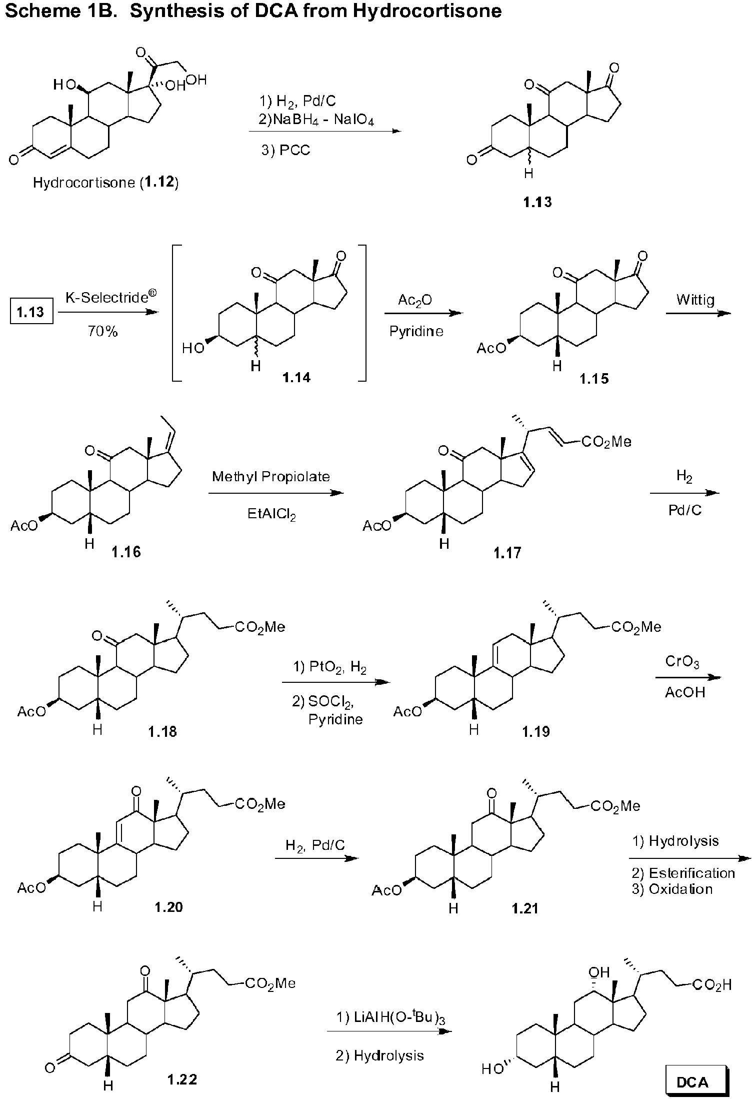

Further transformations of Compounds 1.10 and 2.1 are shown in Scheme 2. First, Compound 1.10 is modified to contain a properly functionalized C ring system identical to that of DCA (Scheme 2). Stereoselective reduction of the C₁₂ carbonyl group yields Compound 2.1 and catalytic hydrogenation of the 9(11) double bond present in Compound 2.1 yields Compound 2.2.

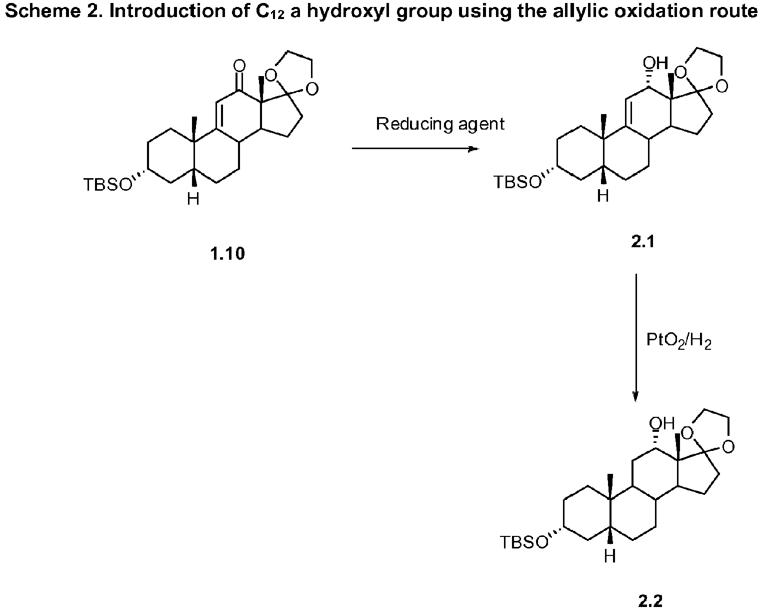

Scheme 3 presents the transformation of epoxide-containing Compound 1.11 to the analogous C₁₂ α-hydroxy steroid Compound 2.2 of Scheme 2.

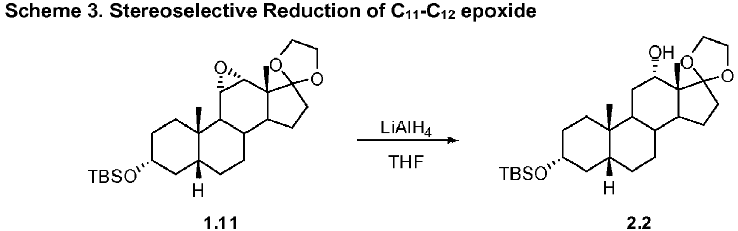

As mentioned above in both of these routes common intermediate compound 2.2 is formed.

The next step in the synthesis of DCA is the modification of the D-ring present in Compound 2.2 such that it contains the carboxylic side chain substituted D ring of DCA (Scheme 4 and Scheme 5).

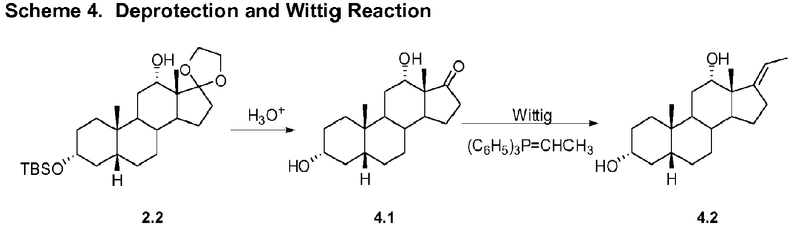

First the C₁₇ ketal and the C₃ silyl ether groups of Compound 2.2 are hydrolyzed. Then the Wittig reaction is performed to yield Compound 4.2. Conversion of Compound 4.2 to Compound 5.1 is carried out via an *ene* reaction. Subsequent catalytic reduction of Compound 5.1 and hydrolysis of the ester yields DCA (Scheme 5).

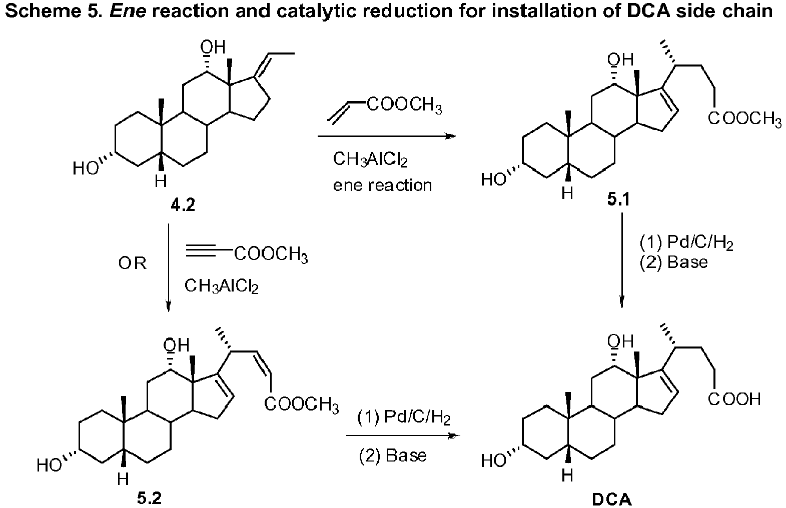

### 2. Synthetic route #2 from Cortisone via Adrenosterone (the i-Steroid, 3,5-cyclosterol route)

Selective ketalization of adrenosterone (Compound 1.2, Scheme 6) at C₁₇, borohydride reduction, mesylation, and buffered hydrolysis yields the i-steroid (3,5-cyclosterol) containing Compound 6.1. Compound 6.1 undergoes 9(11)*-ene* formation (conversion of Compound 6.1 to Compound 6.2, Scheme 6) and allylic oxidation (conversion of Compound 6.2 to Compound 6.3, Scheme 6) followed by carbonyl group reduction to yield Compound 6.4. Hydrolysis of the i-sterol and hydrogenation yields Compound 6.5, which can be converted to DCA by synthetic methods presented above in Synthetic Route #1.

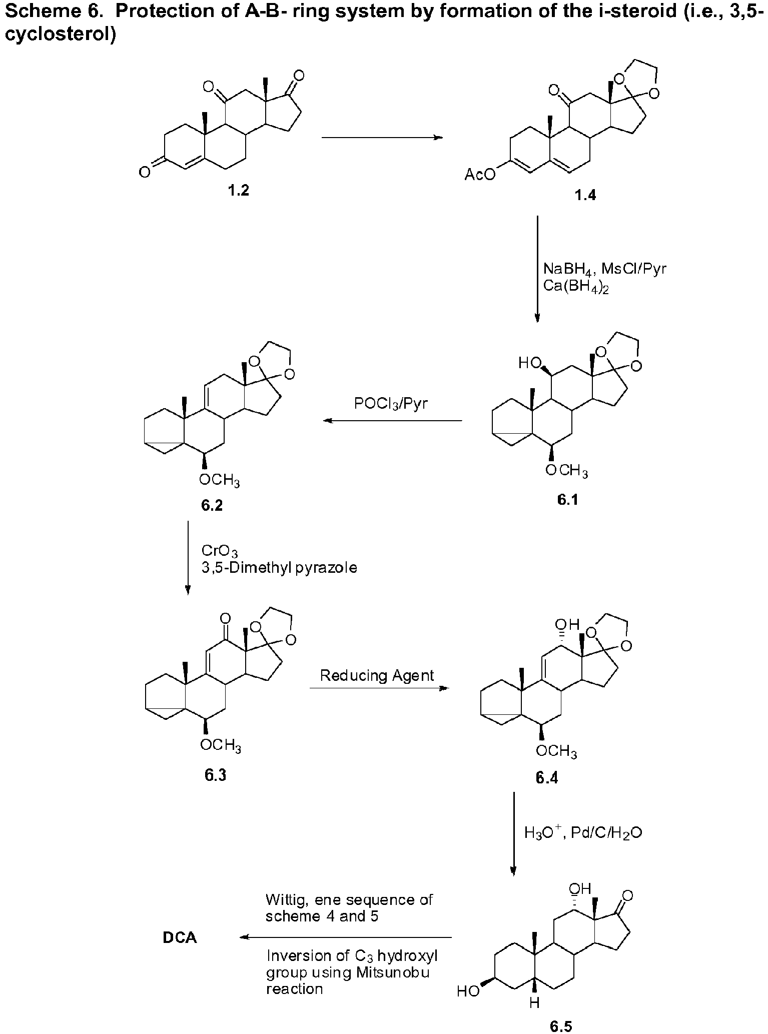

### 3. Synthetic route #3 from Hecogenin

Hecogenin (Compound 7.1, Scheme 7) is a plant sterol found abundantly in Mexican yams and other plants of the Agave species. The central advantage of hecogenin as a starting material for DCA synthesis is that it possesses a C₁₂ oxygen functionality as is present in DCA.

The first step in the synthetic route starting from hecogenin is the stereoselective reduction of the C₁₂ carbonyl group in hecogenin (Compound 7.1) to the requisite C₁₂-α configuration (conversion of Compound 7.1 to Compound 7.2). Then the 3-β-ol, 5α-AB ring system is converted to the 3 α-ol, 5β-AB (Conversion of Compound 7.1, to Compound 7.2, to Compound 7.3) ring system (Scheme 7). The well-known Marker degradation (Marker, R.E., Rohrmann, E., Sterols. LXIX. Oxidation Products of Sarsasapogenin. Sarsasapogenoic Acid and Related Substances, J. Am. Chem. Soc., 61 (8): p. 2072-2077 (1939)) follows the conversion of Compound 7.2 to Compound 7.3 to yield Compound 7.4. Installation of the D-ring side chain (Scheme 8) into Compound 8.2 is achieved via methods shown in Schemes 4 and 5. The requisite C₁₇ ketone in Compound 8.2 is formed by ozonolysis of the enol acetate of Compound 8.1 (Scheme 8). DCA is then prepared from 8.2 in a similar manner as in Scheme 5 using the olefination and ene reaction sequence. Alternative routes starting from starting from hecogenin are shown in Schemes 9 and 10.

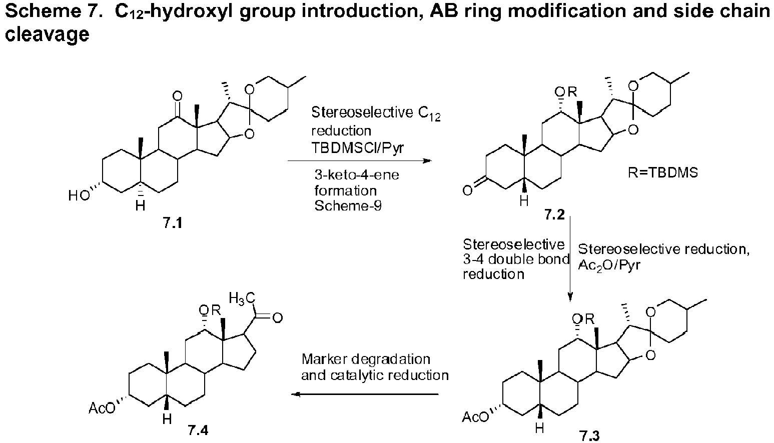

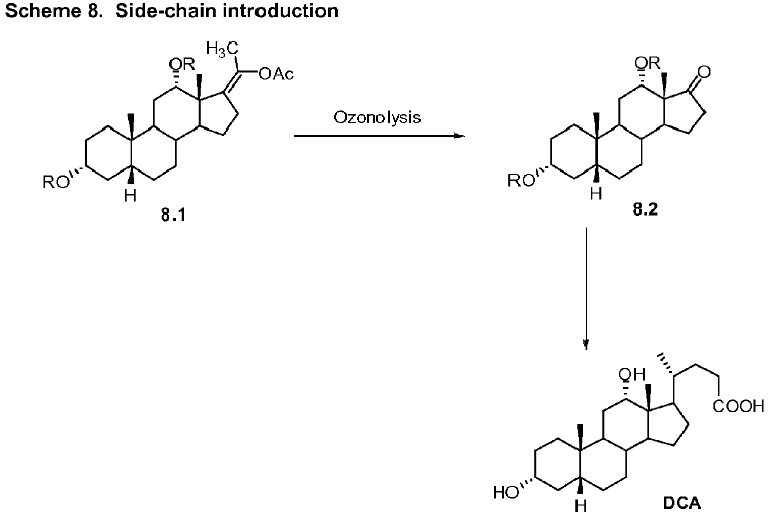

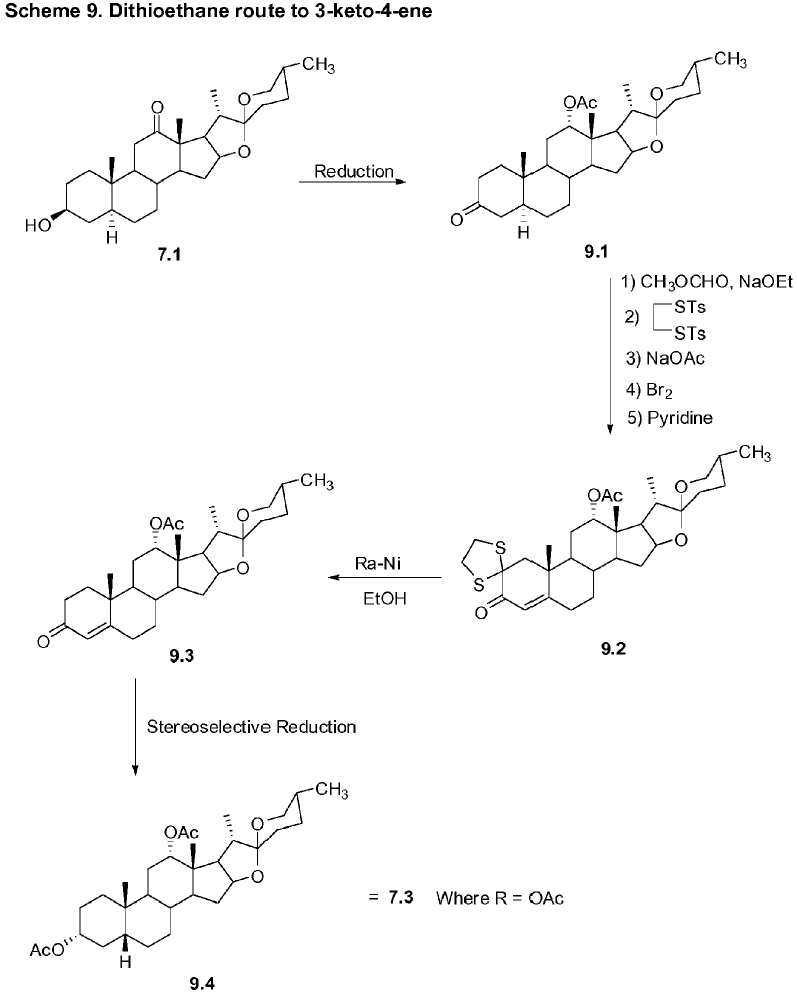

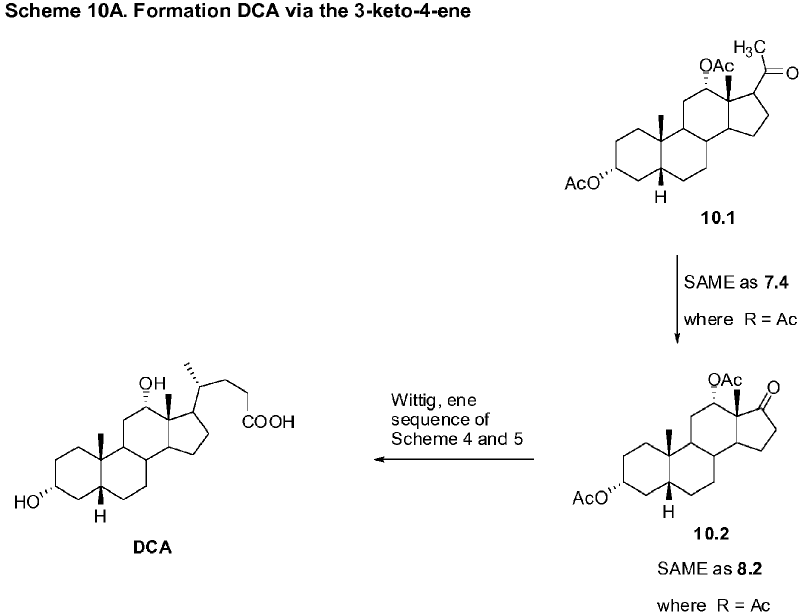

### 4. Synthetic route #4 from Sapogenins

Sapogenins are derived from the hydrolysis of the saccharides and disaccharides attached to the C₃ hydroxyl group of the saponins (i.e., steroid glycosides). These are widely occurring plant products. Saponin occurs in nature as a spiroketal structure as shown below. Also Compound 10.3 can be formed from tigogenin, diosgenin, chlorogenin, smilagenin and hecogenin (Compound 7.1). We believe that DCA could be synthesized from each of these, namely, tigogenin, diosgenin, chlorogenin, smilagenin and hecogenin (Compound 7.1). (Y. Mazur, N. Danieli and Franz Sondheimer, J. Am. Chem. Soc.; 82, 5809 (1960)).

Since we could synthesize DCA from hecogenin, we recognize that any of the above sapogenins could, likewise, serve as a starting material for DCA synthesis.

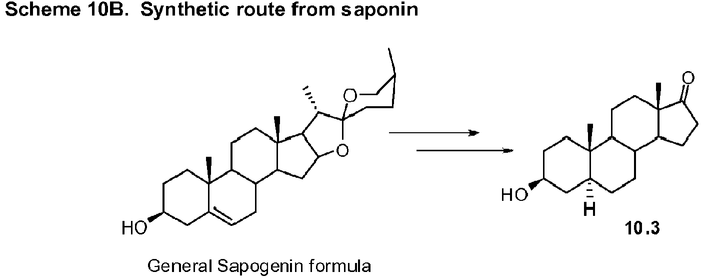

### 5. Synthetic route #5 from Stigmasterol

Stigmasterol (Compound 11.1) is a widely available plant sterol. As a starting material for the DCA synthesis, it has an advantage in that it contains a functionalized AB ring system and a readily cleavable side chain moiety. It has the disadvantage in that it lacks the required functionality in the C ring essential for DCA synthesis.

In this synthetic route, the stigmasterol (Compound 11.1) AB ring is protected by i-steroid formation followed by ozonolysis to yield a side-chain at C₁₇ installed and reduced to the C₂₄-ol as a masked form of the carboxyl group (Scheme 11). The subsequent steps generate an allylic position at C₁₂ (Scheme 12). The B-ring diene formation and mercuric acetate oxidation are known processes and catalytic reduction of the B ring system yields an intermediate common with previous routes described above. However, in contrast to other routes, the side chain is already present. Allylic oxidation (conversion of Compound 12.4 to Compound 1.20) and stereoselective reduction (conversion of Compound 1.20 to Compound 1.21) followed by previously discussed steps, yields a product which is converted to DCA.

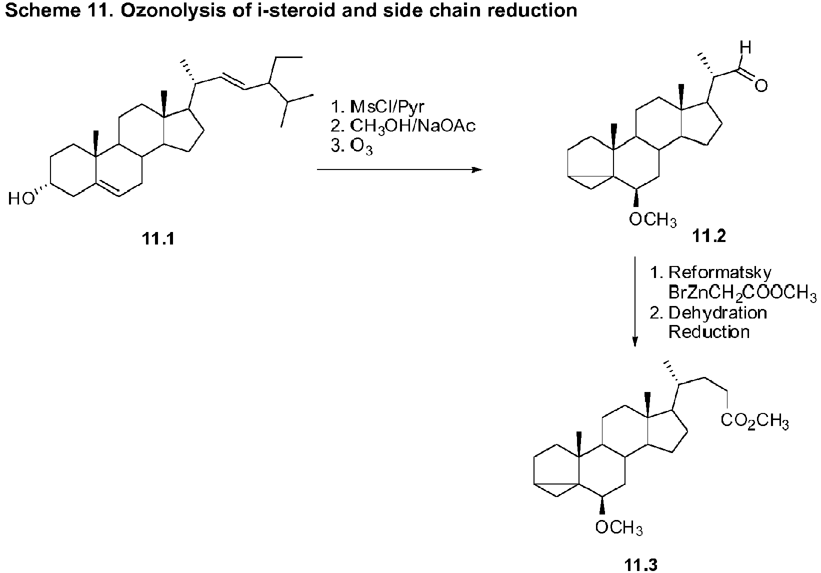

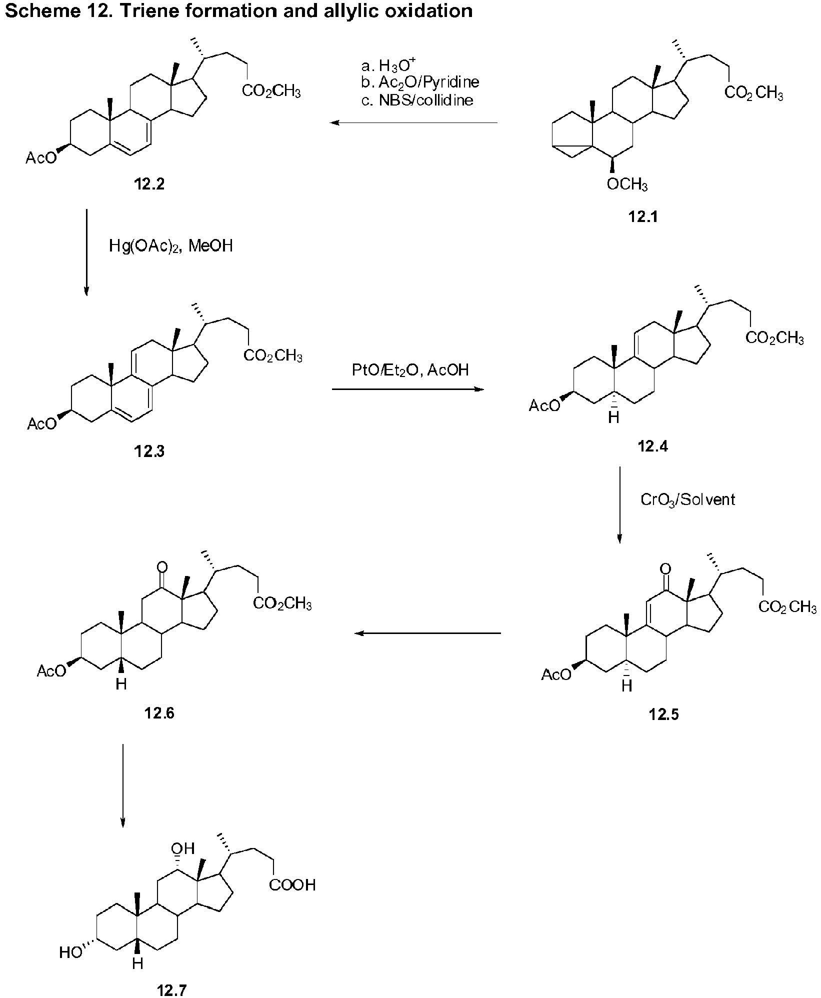

A variation of the stigmasterol route uses the Diels-Alder protection of the B-ring diene. This is advantageous because it isolates the 9(11) double bond to prevent possible interference during the allylic oxidation steps (Scheme 13).

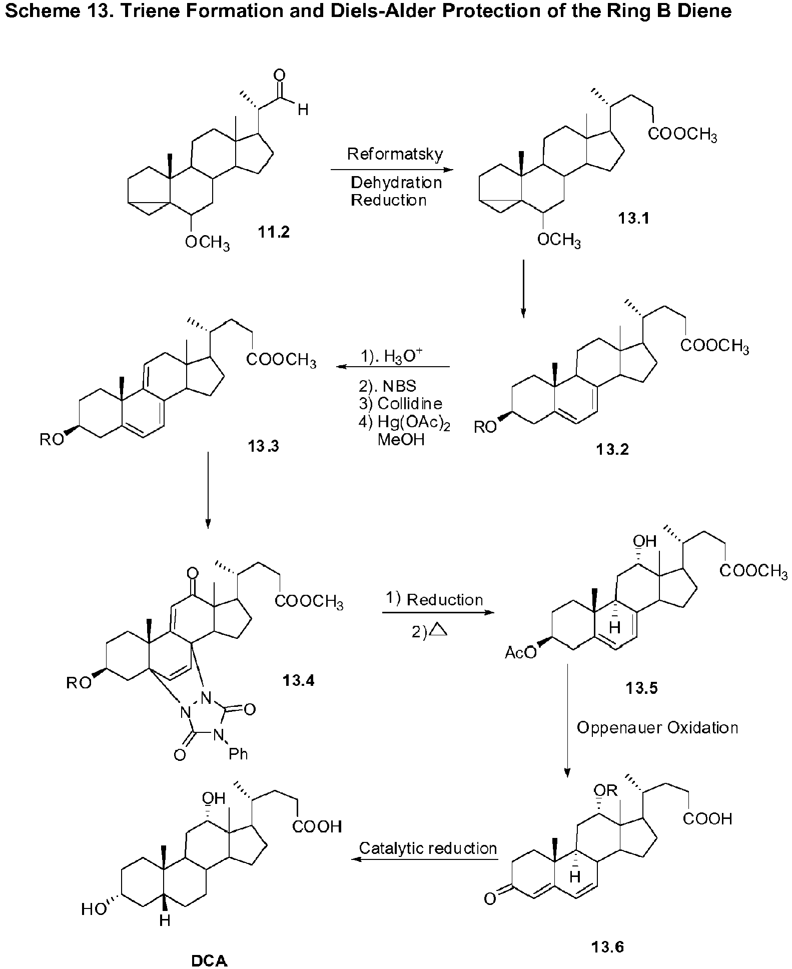

### 6. Synthetic route #6 from Ergosterol

Ergosterol (Compound 14.1) is a readily available starting material and can be used to prepare DCA by adaptation of the procedures set forth in this application. Allylic oxidation offers a facile route to C₁₂ oxygen functionality (Scheme 14). This route has the advantage of starting with the ring B diene. It is convergent with the stigmasterol route.

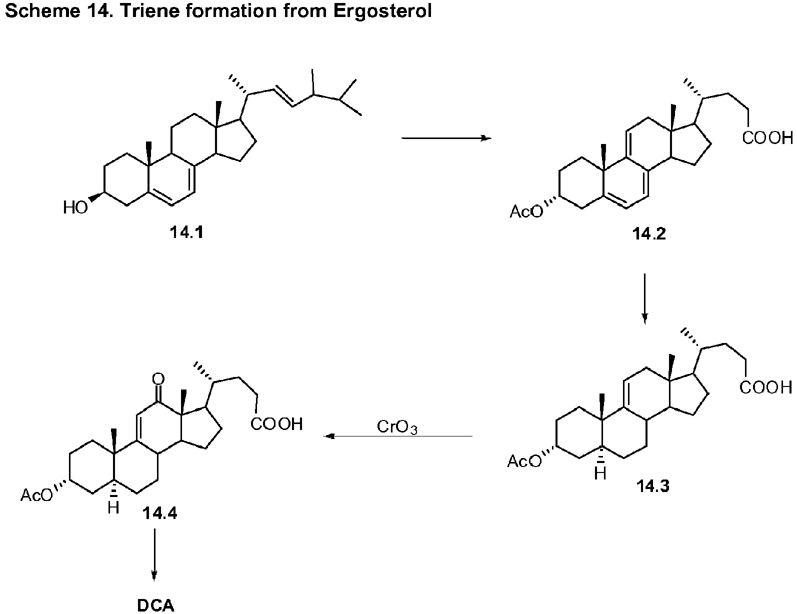

The compounds of preferred embodiments can be prepared from readily available starting materials using the following general methods and procedures. It will be appreciated that where typical or preferred process conditions (i.e., reaction temperatures, times, mole ratios of reactants, solvents, pressures, etc.) are given, other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. Suitable protecting groups for various functional groups as well as suitable conditions for protecting and deprotecting particular functional groups are well known in the art. For example, numerous protecting groups are described in T. W. Greene and G. M. Wuts, Protecting Groups in Organic Synthesis, Third Edition, Wiley, New York, 1999, and references cited therein.

The starting materials and reagents for the reactions described herein are generally known compounds or can be prepared by known procedures or obvious modifications thereof. For example, many of the starting materials and reagents are available from commercial suppliers such as Aldrich Chemical Co. (Milwaukee, Wisconsin, USA), Bachem (Torrance, California, USA), Emka-Chem or Sigma (St. Louis, Missouri, USA). Others may be prepared by procedures, or obvious modifications thereof, described in standard reference texts such as Fieser and Fieser's Reagents for Organic Synthesis, Volumes 1-15 (John Wiley and Sons, 1991), Rodd's Chemistry of Carbon Compounds, Volumes 1-5 and Supplementals (Elsevier Science Publishers, 1989), Organic Reactions, Volumes 1-40 (John Wiley and Sons, 1991), March's Advanced Organic Chemistry, (John Wiley and Sons, 4th Edition), and Larock's Comprehensive Organic Transformations (VCH Publishers Inc., 1989).

### Examples

The various starting materials, intermediates, and compounds of the preferred embodiments may be isolated and purified where appropriate using conventional techniques such as precipitation, filtration, crystallization, evaporation, distillation, and chromatography. Characterization of these compounds may be performed using conventional methods such as by melting point, mass spectrum, nuclear magnetic resonance, and various other spectroscopic analyses.

Exemplary embodiments of steps for performing the synthesis of products in Synthetic Route #1, Scheme 1B is described in greater detail *infra.* Table 3 describes abbreviations used to express various compounds/moieties/apparatus/procedure/property in the exemplary reaction schemes and synthetic routes described in the following examples and throughout the specification.

**Table 3**

| | |
|---|---|
| AcOH | Acetic acid |
| CAN or CH₃CN | Acetonitrile |
| Ac₂O | Acetic anhydride |
| AcCl | Acetyl chloride |
| NH₄Cl | Ammonium chloride |
| bd | Broad doublet |
| CHCl₃ | Chloroform |
| CrO₃ | Chromium trioxide |
| Conc. | concentrated |
| DCA | Deoxycholic acid |
| DCM (CH₂Cl₂) | Dichloromethane |
| DMAP | 4-Dimethylaminopyridine |
| DMF | *N, N*-Dimethylformamide |
| DMSO | Dimethyl sulfoxide |
| d | Doublet |
| dd | Double doublet |
| dt | Double triplet |
| EtOH | Ethanol |
| EtOAc | Ethyl acetate |
| EtAlCl₂ | Ethyl aluminum dichloride |
| g | Gram |
| Hz | Hertz |
| HPLC | High pressure liquid chromatography |
| HCl | Hydrochloric acid |
| LAH | Lithium aluminum hydride |
| LiAl(O^{t}Bu)₃H | Lithium tri-*tert*-butoxyaluminum hydride |
| LiOH | Lithium hydroxide |
| MgSO₄ | Magnesium sulfate |
| MHz | Megahertz |
| MeOH | Methanol |
| □L | microliter |
| mmol | millimole |
| mL | milliliter |
| mm | millimeter |
| min | minute |
| mol | mole |
| m | multiplet |
| Obs | Observed |
| Pd/C | Palladium on carbon |
| HClO₄ | Perchloric acid |
| PtO₂ | Platinum oxide |
| KBr | Potassium bromide |
| *K*-O^{t}Bu | Potassium *tert*-butoxide |
| PCC | Pyridinium chlorochromate |
| q | quartet |
| Rep | Reported |
| s | singlet |
| NaHCO₃ | Sodium bicarbonate |
| NaBH₄ | Sodium borohydride |
| NaOH | Sodium hydroxide |
| Na₂SO₄ | Sodium sulfate |
| NalO₄ | Sodium periodate |
| H₂SO₄ | Sulfuric acid |
| THF | Tetrahydrofuran |
| SOCl₂ | Thionyl chloride |
| TEA | Triethylamine |
| TFA | Trifluoroacetic acid |
| TLC | Thin layer chromatography |
| Wt | Weight |

**General:** Manipulations of oxygen- and moisture-sensitive materials are conducted with two-necked flame dried flasks under an argon atmosphere. Column chromatography is performed using SE-Make silica gel (60-120 Mesh), Spectrochem silica gel (230-400 Mesh) or aluminium oxide 90-neutral. (SD-Fine Chem. Ltd., India). Analytical thin layer chromatography (TLC) was performed on Merck Kieselgel 60 F₂₅₄ (0.25 mm) plates (Merck & Co., Whitehouse Station, NJ). Visualization of spots was detected either by UV light (254 nm) lamp or by charring with a solution of sulfuric acid (5%) and p-anisaldehyde (3%) in ethanol.

**Apparatus:** Analysis of the compounds and products of the reaction schemes and synthetic routes described herein may be performed on the apparatus and equipment described *infra.*

### Nuclear Magnetic Resonance (NMR)

Proton and carbon nuclear magnetic resonance spectra (¹H NMR and ¹³C NMR) are recorded on a Varian Mercury-Gemini 200 (¹H NMR, 200 MHz; ¹³C NMR, 50 MHz) or a Varian Mercury-Inova 500 (¹H NMR, 500 MHz; ¹³C NMR, 125 MHz) (Varian, Inc., Palo Alto, CA) spectrometer with solvent resonances as the internal standards (¹H NMR, CHCl₃ at 7.26 ppm or DMSO at 2.5 ppm and DMSO-H₂O at 3.33 ppm; ¹³C NMR, CDCl₃ at 77.0 ppm or DMSO at 39.5 ppm). ¹H NMR data are reported as follows: chemical shift (δ, ppm), multiplicity (s = singlet, d = doublet, t = triplet, q = quartet, br = broad, m = multiplet), coupling constants (Hz), and integration.

### Infrared Spectroscopy

Infrared spectra (FT-IR) are run on a JASCO-460⁺ model (Jasco, Inc., Easton, MD). Mass spectra are obtained with a Perkin Elmer, API-2000 spectrometer (Perkin Elmer, Inc., Waltham, MA) using ES⁺ mode.

### Melting Point

Melting points were determined using a LAB-INDIA melting point measuring apparatus (Labindia Instruments Pvt. Ltd., India) and are uncorrected.

### High Pressure Liquid Chromatography

HPLC chromatograms were recorded using a SHIMADZU-2010 model with a PDA detector (Shimadza Corp., Japan).

### Optical Activity

Specific optical rotations ([α]_{D}) are determined employing a JASCO-1020 at 589 nm (Jasco, Inc., Easton, MD) and are uncorrected.

**Chemicals:** Unless otherwise noted, commercially available reagents are used without purification. Diethyl ether and THF are distilled from sodium/benzophenone ketyl. Anhydrous DMF, DCM, pentane and hexane are obtained by distillation from CaH₂.

### Example 1

### Preparation of Androstane-3,11,17-trione (1.13)

10% of Pd/C (2.5 g, 5 wt %) is added to a solution of hydrocortisone (Compound 1.12) (50.0 g, 138.12 mmol) in DMF (250 mL). The resulting slurry is hydrogenated in a Parr apparatus (50 psi) for 12 h. Upon complete disappearance of starting material, as evidenced by TLC, the crude reaction mixture is filtered through a small plug of Celite, and the solvent is removed under vacuum. Crude product (48.0 g) is obtained as a colorless solid.

NaBH₄ (2.1 g, 55.3 mmol) is added to a solution of the above crude product (48.0g, 131.86 mmol) in EtOH (500 mL) and CH₂Cl₂ (500 mL). After 1 hr, acetone (50 mL) and water (150 mL) are added, followed by NalO₄ (70.5 g, 329.6 mmol). The mixture is stirred at room temperature overnight.

Distilled water (500 mL) is added and the mixture is extracted with ethyl acetate (3 × 250 mL). The ethyl acetate layer is flushed through a silica-gel plug and the solvent is evaporated to yield 38 g as a colorless solid. The crude product is oxidized further without purification.

PCC (40.4 g, 187.5 mmol) is added to a solution of the above crude product in CH₂Cl₂ (400 mL) in 3 equal portions over 30 minutes. The resulting reaction mixture is stirred at room temperature for about 3-4 h. Upon completion of the reaction, as monitored by TLC, the crude reaction mixture is filtered sequentially through pads of Celite and silica gel and the crude material is purified by column chromatography [59(W)×700(L) mm, 60-120 Mesh silica, 150 g], eluting with ethyl acetate/hexane (3:10) [50 mL fractions, 10 mL/min elution, monitored by TLC with p-anisaldehyde charring; R_{f} for Compound 1.13 = 0.37 and R_{f} for Compound 1.12 = 0.05 in EtOAc/Hexane (1 : 1)] to provide the diastereomeric Compound 1.13 (33.0 g, 79% yield) as a colorless solid.

The obtained crude material was purified by preparative HPLC using a Phenomenex Lunov C18 column (250 × 30.0 mm, 10µ) and isocratic elution with CH₃CN:H₂O (12:13) with a 25 mL/min flow rate in 15 mL fractions. The preparative HPLC is only used for purification, but not for analysis. Table 4 describes the measured properties of the product.

**Table 4**

| | |
|---|---|
| ¹H NMR (500 MHz, CDCl₃) | δ = 2.76 (dt, *J* = 4.0, 15.0 Hz, 1H), 2.62-2.35 (m, 5H), 2.33-2.24 (m, 1 H), 2.23-2.05 (m, 4H), 2.02-1.88 (m, 3H), 1.81 (bd, *J* = 14.0 Hz, 2H), 1.72-1.61 (m, 1H), 1.57-1.48 (m, 1H), 1.47-1.32 (m, 2H), 1.26 (s, 3H), 0.86 (s, 3H) |
| ¹³C NMR (125 MHz, CDCl₃) | δ = 216.9, 211.8, 208.4, 52.3, 50.3, 50.2, 50.0, 44.5, 41.9, 37.1, 36.0, 35.9, 35.8, 34.3, 25.6, 25.0, 22.2, 21.3, 14.5 |
| Mass (m/z) | 303.2 [M⁺ + 1], 320.1 [M⁺ + 18] |
| IR (KBr) | 3443, 2916, 1729, 1705, 1466, 1379, 1044 cm⁻¹ |
| m.p. | 128.9-131 °C (from CH₂Cl₂/Hexane) (observed); 128-131 °C (Rep. E.Caspi J. Org. Chem., 24, 669 (1959)) |
| [α]_{D} | +139 (c = 1 in CHCl₃). |
| HPLC purity | 98.6%, ret. time = 16.61, (Hypersil BDS C18; 250 × 4.6 mm, 5u), ACN: 5 mM TEA pH-2.5 with HClO₄ (Gradient), absorbance at 205 nm |

### Example 2

### 3β-Hydroxy-androstane-11,17-dione (1.14)

*K*-selectride® (98.39 mL, 98.01 mmol, 1 M solution in THF) is added to a solution of Compound 1.13 (33.0 g, 109.27 mmol) in THF (330 mL) over 15 minutes under an inert atmosphere at -78 °C and is stirred for about 3-4 h at -78 °C. The reaction mixture is quenched with aqueous NaOH solution (2M, 70 mL). The crude reaction mixture is diluted with ethyl acetate (500 mL) and the organic layer is washed with water (3 × 75 mL), saturated brine solution (100 mL) and dried over MgSO₄ (75 g). The solvent is removed under vacuum to afford 33 g of crude material. The crude product is subjected to acetylation without purification.

### Purification of Crude Material

The crude material is purified by column chromatography [29(W)×600(L) mm, 230-400 Mesh silica, 200 g], eluting with ethyl acetate/hexane (1 : 4) [25 mL fractions, 5 mL/min elution, monitored by TLC with p-anisaldehyde charring; R_{f} for Compound 1.14 = 0.3 and R_{f} for Compound 1.13 = 0.37 in EtOAc/Hexane (1 : 1)] to afford Compound 1.14. Table 5 describes the measured properties of the product.

**Table 5**

| | |
|---|---|
| ¹H NMR (500 MHz, CDCl₃) | δ = 4.08 (s , 1H ), 2.53 (q, *J* = 9.0 Hz, 1H), 2.42 (d, *J* = 13.0 Hz, 1H), 2.34-2.21 (m, 3H), 2.11-2.04 (m, 1H), 1.98-1.91 (m, 3H), 1.88-1.59 (m, 6H), 1.57-1.26 (m, 6H), 1.21 (s, 3H), 0.82 (s, 3H) |
| ¹³C NMR (125 MHz, CDCl₃) | δ = 217.4, 209.1, 66.3, 51.6, 50.6, 50.5, 37.1, 36.2, 35.9, 34.8, 33.5, 28.8, 28.5, 25.7, 25.6, 23.6, 21.5, 14.5 |
| Mass (m/z) | 305.0 [M⁺ + 1], 322.0 [M⁺ + 18] |
| IR (KBr) | 3519, 2928, 1735, 1697, 1454, 1379 cm⁻¹ |
| m.p. | 176.6-180.5 °C |
| [α]_{D} | +125 (c = 1 in CHCl₃) |

### Example 3

### 3β-Hydroxyandrostane-11,17-dione acetate (1.15)

Acetic anhydride (16.6 g, 162.8 mmol) is added to a solution of Compound 1.14 (33.0 g, 108.55 mmol) in pyridine (150 mL) at 0 °C under an inert atmosphere. The resulting reaction mixture is stirred overnight at ambient temperature. Upon completion of the reaction, as evidenced by TLC, pyridine and remaining acetic anhydride are removed under vacuum. The crude residue is diluted with ethyl acetate (500 mL) and washed with water (3 × 150 mL), saturated brine solution (100 mL) and dried over MgSO₄ (75 g). The solvent is evaporated under vacuum and the crude material is purified by column chromatography [59(W)×800(L) mm, 60-120 Mesh silica, 150 g], eluting with ethyl acetate/hexane (1:10) [25 mL fractions, 10 mL/min elution, monitored by TLC with p-anisaldehyde charring; R_{f} for Compound 1.15 = 0.38 and R_{f} for Compound 1.14 = 0.1 in EtOAc/Hexane (3 : 7)] to afford Compound 1.15 (19.0 g, 66.4 % yield) as a colorless solid. Table 6 describes the measured properties of the product.

**Table 6**

| | |
|---|---|
| ¹H NMR (500 MHz, CDCl₃): | δ = 5.03 (s, 1H), 2.53 (dd, *J* = 9.5, 19.0 Hz, 1H), 2.42 (d, *J* = 10.0 Hz, 1H), 2.36-2.31 (m, 3H), 2.25 (dd, *J* = 9.5, 19.0 Hz, 1H), 2.10-2.06 (m, 1H), 2.04 (s, 3H), 1.96-1.91 (m, 3H), 1.81-1.69 (m, 2H), 1.63-1.57 (m, 3H), 1.50 (dd, *J* = 3.0, 14.5 Hz, 1H), 1.36 (d, *J* = 9.5 Hz, 3H), 1.27-1.22 (m, 1H), 1.20 (s, 3H), 0.82 (s, 3H) |
| ¹³C NMR (125 MHz, CDCl₃) | δ = 217.2, 208.9, 170.4, 69.7, 51.5, 50.5, 50.4, 37.9, 36.1, 35.9, 34.5, 30.6, 29.6, 29.5, 25.5, 25.4, 25.3, 23.4, 21.4, 21.3, 14.5 |
| Mass (m/z) | 347.1 [M⁺ + 1], 364.1 [M⁺ + 18] |
| IR (KBr) | 3455, 2927, 1737.6, 1720.2, 1707.7, 1259, 1244 cm⁻¹ |
| m.p. | 156-158 °C |
| [α]_{D} | 116 (c = 1 in CHCl₃) |

### Example 4

### (Z)-3β-Hydroxy-5(β-preg-17(20)-ene-11-one acetate (1.16)

Potassium *tert*-butoxide (159.28 mL, 159.2 mmol, 1 M solution in THF) is added to a solution of ethyltriphenylphosphonium bromide (61.16 g, 164.8 mmol) in THF (150 mL) is added drop wise over 1 h under an inert atmosphere at -5 °C. The resulting dark pink colored reaction mixture is warmed to 10-15 °C and stirred for an additional 1 h at the same temperature. A solution of Compound 55 (19.0 g, 54.9 mmol) in THF (50 mL) is introduced slowly to the above Wittig ylide suspension at -5 °C. The solution is stirred for an additional 10-20 minutes and the reaction mixture is allowed to warm to ambient temperature slowly. Stirring is continued for about 3-4 h. Upon complete disappearance of starting material, as evidenced by TLC, the reaction mixture is quenched with saturated aqueous NH₄Cl solution (75 mL). The aqueous layer is extracted with EtOAc (2 × 150 mL) and the combined organic extracts are washed with saturated brine solution (100 mL) and dried over MgSO₄ (75 g). The solvent is removed under vacuum and the crude material is purified by column chromatography [49(W)×600(L) mm, 60-120 Mesh silica, 300 g] eluting with ethyl acetate/hexane (1 : 20) [25 mL fractions, 10 mL/min elution, monitored by TLC with p-anisaldehyde charring; R_{f} for Compound 1.16 = 0.54 and R_{f} for Compound 1.15 = 0.06 in EtOAc/Hexane (1 : 6)] to afford Compound 1.16 (15.5 g, 78.8% yield) as a thick colorless liquid, which solidified slowly after 1-2 days at 0 °C. Table 7 describes the measured properties of the product.

**Table 7**

| | |
|---|---|
| ¹H NMR (500 MHz, CDCl₃) | δ = 5.20-5.15 (m, 1H), 5.03 (s, 1H), 2.86 (d, *J* = 10.0 Hz, 1H), 2.60 (d, *J* = 10.0 Hz, 1H), 2.46-2.28 (m, 5H), 2.01 (s, 3H), 1.94-1.62 (m, 5H), 1.60-1.52 (m, 6H), 1.48-1.45 (m, 1H), 1.41-1.36 (m, 4H), 1.20 (s, 3H), 0.82 (s, 3H) |
| ¹³C NMR (125 MHz, CDCl₃) | δ = 210.9, 170.5, 147.2, 114.7, 70.0, 56.1, 55.6,51.5, 47.4, 37.9, 35.9, 34.4, 31.6, 30.7, 29.8, 26.4, 25.9, 25.5, 24.0, 23.5, 21.3, 17.9, 12.8 |
| Mass (m/z) | 359.2 [M⁺ + 1], 376.2 [M⁺ + 18] |
| IR (CHCl₃) | 3421, 2928, 1734, 1704, 1377, 1243 cm⁻¹ |
| m.p. | 88.5-91.2 °C |
| [α]_{D} | +30 (c = 1 in CHCl₃) |

### Example 5

### Methyl (E)-3β-hydroxy-5β-chola-16(17),22(23)-diene-24-oate acetate (1.17)

Methyl propiolate (9.68 g, 114.95 mmol) is added to a solution of Compound 1.16 (16.5 g, 46 mmol) in CH₂Cl₂ (220 mL) 0 °C. The reaction mixture is warmed to ambient temperature and stirred for 1 h under an inert atmosphere. Ethyl aluminum dichloride (17.5 g, 137.8 mmol) is introduced to the above mixture at 0 °C drop wise and the resulting reaction mass is again warmed to ambient temperature and stirred overnight. Upon completion of the reaction, as evidenced by TLC, the crude reaction mixture is quenched with ice-water (100 mL) and the aqueous layer is extracted with EtOAc (3 × 150 mL). The combined organic layer is washed with saturated brine solution (100 mL) and dried over MgSO₄ (50 g). The solvent is removed under vacuum and the crude material is purified by column chromatography [49(W)×600(L) mm, 60-120 Mesh silica, 300 g] eluting with ethyl acetate/hexane (1 : 7) [15 mL fractions, 10 mL/min elution, monitored by TLC and detected with either by UV light (254 nm) lamp or *p*-anisaldehyde charring; R_{f} for Compound 1.17 = 0.36 and R_{f} for Compound 1.16 = 0.54 in EtOAc/Hexane (1 : 6)] to afford Compound 1.17 (16 g, 79% yield) as a colorless semi solid. Table 8 describes the measured properties of the product.

**Table 8**

| | |
|---|---|
| ¹H NMR (500 MHz, CDCl₃) | δ = 6.89 (dd, *J* = 8.0, 16 Hz, 1H), 5.81 (d, *J* = 15 Hz, 1H), 5.48 (s, 1H), 5.03 (s, 1H), 3.73 (s, 3H), 2.95 (t, *J* = 6.5 Hz, 1H), 2.45-2.36 (m, 2H), 2.30-2.17 (m, 2H), 2.04 (s, 3H), 2.00-1.79 (m, 5H), 1.58 (s, 3H), 1.49-1.18 (m, 9H), 1.16 (s, 3H), 0.70 (s, 3H) |
| ¹³C NMR (125 MHz, CDCl₃) | δ = 209.7, 170.1, 166.6, 154.4, 152.3, 124.5, 119.1, 69.7, 56.3, 53.8, 52.3, 51.1, 49.8, 37.9, 35.7, 35.0, 34.4, 30.5, 30.4, 29.6, 26.2, 25.6, 25.2, 23.3, 21.1, 19.2, 17.3 |
| Mass (m/z) | 443.0 [M⁺ + 1], 460.1 [M⁺ + 18] |
| IR (CHCl₃) | 3438, 2930, 1729, 1706, 1653, 1448, 1435, 1243, 1022 cm⁻¹ |
| [α]_{D} | +59 (c = 1 in CHCl₃) |
| HPLC purity | 94.4%; ret. time = 28.86, (Zorbax SB, C18; 250 × 4.6 mm, 5u), ACN: 5 mM TEA pH-2.5 with HClO₄ (Gradient); absorbance at 205 nm |

### Example 6

### Methyl 3β-hydroxy-5β-cholan-11-one-24-oate acetate (1.18)

10% Pd/C (2.9 g, 20 wt%) is added to a solution of Compound 1.17 (14.5 g, 32.8 mmol) in EtOAc (150 mL). The resulting slurry is hydrogenated in a Parr apparatus (50 psi) for 12 h. Upon complete disappearance of starting material, as evidenced by TLC [R_{f} for Compound 1.18 = 0.43 and R_{f} for Compound 1.17 = 0.43 in EtOAc/Hexane (1 : 3); however only Compound 1.17 is UV active from the conjugated ester chromophore], the crude reaction mixture is filtered through a small plug of Celite and the solvent is removed under vacuum to afford Compound 1.18 (14 g, 95.7% yield) as a colorless solid. Table 9 describes the measured properties of the product.

**Table 9**

| | |
|---|---|
| ¹H NMR (500 MHz, CDCl₃) | δ = 5.03 (s, 1H), 3.73 (s, 3H), 2.56 (d, *J* = 10 Hz, 1H), 2.38-2.19 (m, 5H), 2.04 (s, 3H), 1.86-1.13 (m, 20H), 1.12 (s, 3H), 0.86 (s, 3H), 0.62 (s, 3H) |
| ¹³C NMR (125 MHz, CDCl₃) | δ = 211.3, 174.3, 170.5, 70.0, 58.3, 55.7, 55.0, 51.4, 50.8, 46.8, 37.9, 36.7, 35.0, 34.3, 30.9, 30.7, 30.7, 29.7, 28.3, 26.6, 25.9, 25.5, 23.6, 23.5, 21.4, 17.9, 12.7 |
| Mass (m/z) | 447.1 [M⁺ + 1], 464.1 [M⁺ + 18] |
| IR (KBr) | 3449, 2927, 1734, 1704, 1381, 1262, 1243 cm⁻¹ |
| m.p. | 174.2-175.7 °C (From CH₂Cl₂/Hexane) (Observed); 174.8-176.2 °C (Reported) |
| [α]_{D} | +39 (c = 1 in CHCl₃) |

### Example 7

### Methyl 3β-hydroxy-5β-chol-9(11)-ene-24-oate acetate (1.19)

PtO₂(5.0 g, 100 wt%) is added to a solution of 1.18 (5.0 g, 11.2 mmol) in EtOAc (75 mL) in the presence of catalytic amount of AcOH (2.0 mL). The resulting slurry is hydrogenated in a Parr apparatus (70 psi) for about 14-16 h. Upon completion of the reaction, the crude mixture is filtered through a small plug of Celite and the solvent is removed under vacuum. The crude product is used for the elimination reaction without further purification.

SOCl₂ (1.98 g, 16.78 mmol) is introduced to a solution of the above crude material in pyridine (100 mL) drop wise at 0 °C. The resulting reaction mixture is warmed to ambient temperature and stirred for about 1 h. Upon completion of the reaction, as evidenced by TLC, pyridine is removed under vacuum. The crude residue is diluted with ethyl acetate (100 mL) and washed with water (2 × 50 mL), saturated brine solution (100 mL) and dried over MgSO₄ (40 g). The solvent is evaporated under vacuum and the crude material is purified by column chromatography [49(W)×600(L) mm, 60-120 Mesh silica, 120 g] eluting with ethyl acetate/hexane (1:10) [10 mL fractions, 5 mL/min elution, monitored by TLC with p-anisaldehyde charring; R_{f} for Compound 1.19 = 0.51 and R_{f} for Compound 1.18 = 0.22 in EtOAc/Hexane (1 : 6)] to afford Compound 1.19 (4.1 g, 85.4% yield) as a colorless solid. Table 10 describes the measured properties of the product.

**Table 10**

| | |
|---|---|
| ¹H NMR (500 MHz, CDCl₃) | δ = 5.33 (s, 1H), 5.03 (s, 1H), 3.66 (s, 3H), 2.37-2.32 (m, 1H), 2.46-2.21 (m, 1H), 2.11-2.04 (m, 1H), 2.03 (s, 3H), 1.99-1.10 (m, 22H), 1.07 (s, 3H), 0.92 (d, *J* = 7.0 Hz, 3H), 0.58 (s, 3H) |
| ¹³C NMR (125 MHz, CDCl₃) | δ = 174.6, 170.6, 140.1, 118.9, 71.2, 56.1, 53.3, 51.3, 42.0, 40.9, 38.9, 37.3, 36.4, 35.2, 31.9, 31.4, 31.0, 30.9, 30.1, 28.2, 26.9, 26.2, 26.1, 25.3, 21.4, 17.9, 11.6 |
| Mass (m/z) | 448.2 [M⁺ + 18] |
| IR (KBr) | 3447, 2935, 1735, 1379, 1261, 1245 cm⁻¹ |
| m.p. | 188.6-191.2 °C (From CH₂Cl₂/Hexane) (Observed); 174-175 °C (Reported) |
| [α]_{D} | + 37 (c = 1 in CHCl₃) |

### Example 8

### Methyl 3β-hydroxy-5β-chol-9(11)-ene-12-one-24-oate acetate (1.20)

CrO₃ (8.0g, 100 wt%, 80.0 mmol) is added to a solution of Compound 1.19 (8.0 g, 18.6 mmol) in AcOH (150 mL). The resulting reaction mixture is heated at 60 °C for about 24-36 h. Upon complete disappearance of the precursor, acetic acid is evaporated under vacuum, and the crude material is dissolved in diethyl ether (400 mL). The organic layer is washed with water (2 × 100 mL), saturated brine solution (100 mL) and dried over MgSO₄ (40 g). The solvent is removed under vacuum and the crude material is purified by column chromatography [49(W)×600(L) mm, 60-120 Mesh silica, 120 g] eluting with ethyl acetate/hexane (1 : 5) [10 mL fractions, 3 mL/min elution, monitored by TLC and detected with UV light (254 nm) lamp; R_{f} for Compound 1.20 = 0.28 and R_{f} for Compound 1.19 = 0.61 in EtOAc/Hexane (1 : 4)] to afford Compound 1.20 (5 g, 60.5% yield) as a colorless solid. Table 11 describes the measured properties of the product.

**Table 11**

| | |
|---|---|
| ¹H NMR (500 MHz, CDCl₃) | δ = 5.72 (s, 1H), 5.04 (s, 1H), 3.66 (s, 3H), 2.41-2.27 (m, 3H), 2.03 (s, 3H), 1.94-1.58 (m, 9H), 1.48-1.30 (m, 11H), 1.21 (s, 3H), 1.02 (d, *J* **=** 6.5 Hz, 3H), 0.91 (s, 3H) |
| ¹³C NMR (500 MHz, CDCl₃) | δ = 205.1, 174.5, 170.4, 164.2, 123.1, 70.3, 53.4, 53.0, 51.3, 47.2, 40.2, 37.7, 37.3, 35.2, 32.1, 31.4, 31.0, 30.6, 30.2, 27.3, 26.5, 25.9, 25.6, 24.1, 21.3, 19.4, 10.6 |
| Mass (m/z) | 445.0 [M⁺ + 1], 462.1 [M⁺ + 18] |
| IR | 3447, 2927, 2361, 2339, 1736, 1678, 1367, 1250 cm⁻¹ |
| m.p. | 185.8-188.1 °C (From CH₂Cl₂/Hexane) |
| [α]_{D} | +62 (c = 1 in CHCl₃) |
| HPLC purity | 94.1%; ret. time = 23.89 (Hypersil BDS C18, 250 × 4.6 mm, 5u, CH₃CN : 5 mM TEA, pH-2.5 with HClO₄ (Gradient); absorbance at 240 nm |

### Example 9

### Methyl 3β-hydroxy-5β-cholane-12-one-24-oate acetate (1.21)

10% Pd/C (30 mg, 10 wt%) is added to a solution of Compound 1.20 (300 mg, 0.675 mmol) in EtOAc (30 mL). The resulting slurry is hydrogenated in a Parr apparatus (50 psi) for about 16 h. Upon complete disappearance of starting material by TLC [R_{f} for Compound 1.21 = 0.44 and R_{f} for Compound 1.20 = 0.44 in EtOAc/Hexane (3 : 7); however only Compound 1.20 is UV active from its enone chromophore; additionally charring of Compound 1.20 is faint but Compound 1.21 is bright], the crude reaction mixture was filtered through a small plug of Celite and the solvent is removed under vacuum to afford Compound 1.21 (270m g, 90% yield) as a colorless solid. Table 12 describes the measured properties of the product.

**Table 12**

| | |
|---|---|
| ¹H NMR (500 MHz, CDCl₃) | δ = 5.04 (s, 1H), 3.64 (s, 3H), 2.52-2.47 (m, 1H), 2.38-2.25 (m, 3H), 2.23-2.03 (m, 2H), 2.02(s, 3H), 1.99-1.71 (m, 8H), 1.49-1.11 (m, 12H), 1.05 (s, 3H), 1.01 (s, 3H), 0.85-0.84 (d, *J* = 7.0 Hz, 3H) |
| ¹³C NMR (125 MHz, CDCl₃) | δ = 214.7, 174.6, 170.5, 70.2, 58.7, 57.5, 51.4, 46.5, 43.7, 38.4, 36.9, 35.7, 35.6, 35.5, 31.3, 30.7, 30.5, 27.5, 26.4, 25.9, 24.8, 24.3, 23.2, 21.4, 18.6, 11.7 |
| Mass (m/z) | 447.0 [M⁺ + 1], 464.0 [M⁺ + 18] |
| IR (KBr) | 3447, 2935, 1735, 1704, 1260, 1241 cm⁻¹ |
| m.p. | 179.6-182.7 °C (From CH₂Cl₂/Hexane) |
| [α]_{D} | +69 (c = 1 in CHCl₃) |

### Example 10

### Methyl 5β-chola-3,12-dione-24-oate (1.22)

NaOH (73 mg, 1.8 mmol) is added to a solution of Compound 2.1 (270 mg, 0.6 mmol) in MeOH (10 mL). The resulting reaction mixture is stirred for about 2 h at ambient temperature. Upon completion of the reaction, as evidenced by TLC, MeOH is removed under vacuum and the crude product is diluted with ethyl acetate (20 mL). The organic layer is washed with saturated brine solution (10 mL) and dried over MgSO₄ (5.0 g). The solvent is removed under vacuum and the crude material is used in the esterification reaction without purification.

SOCl₂ (0.1 mL, 1.35 mmol) is added drop-wise to a solution of the above crude material in MeOH (10 mL) 0 °C. The resulting reaction mixture is stirred at ambient temperature for about 1 h. Upon completion of the reaction, MeOH is removed under vacuum. The crude reaction mixture is diluted with EtOAc (30 mL), and the organic layer is washed with water (3 × 10 mL), saturated brine solution (15 mL) and dried over MgSO₄ (5 g). The solvent is evaporated under vacuum and the crude product is used for the oxidation reaction without purification.

PCC (1.0 g, 4.6 mmol) is introduced in 3 equal portions to a solution of the obtained ester in CH₂Cl₂ (25 mL) over about 5 minutes. The resulting reaction mixture is stirred at ambient temperature for about 3-4 h. Upon completion of the reaction, as evidenced by TLC, the crude reaction mixture is filtered through a pad of Celite. The solvent is removed under vacuum and the crude material is purified by column chromatography [19(W)×400(L) mm, 60-120 Mesh silica, 45 g] eluting with ethyl acetate/hexane (1 : 6) [10 mL fractions, 5 mL/min elution, monitored by TLC with p-anisaldehyde charring; R_{f} for Compound 1.22 = 0.57 and R_{f} for Compound 1.21 = 0.71 in EtOAc/Hexane (2 : 3)] to afford Compound 1.22 (170 mg, 70.8% yield) as a colorless solid. Table 13 describes the measured properties of the product.

**Table 13**

| | |
|---|---|
| ¹H NMR (500 MHz, CDCl₃) | δ = 3.66 (s, 3H), 2.64-2.57 (m, 2H), 2.55-2.19 (m, 4H), 2.17-2.02 (m, 3H), 1.99-1.21 (m, 17H), 1.11 (s, 3H), 1.05 (s, 3H), 0.86 (d, *J* = 10.0 Hz, 3H) |
| ¹³C NMR (125 MHz, CDCl₃) | δ = 213.9, 211.8, 174.5, 58.5, 57.5, 51.4, 46.5, 44.2, 43.7, 42.1, 38.3, 36.9, 36.8, 35.6, 35.4, 31.3, 30.5, 27.4, 26.6, 25.4, 24.3, 22.1, 18.5, 11.7 |
| Mass (m/z) | 403.1 [M⁺ + 1], 420.2 [M⁺ + 18] |
| IR (KBr) | 3457, 2925, 1737, 1708, 1216, 1176 cm⁻¹ |
| m.p. | 133.7-135.9 °C (From CH₂Cl₂/Hexane) (Obs); 136.5-137.5 °C (Rep) |
| [α]_{D} | +79 (c = 1 in CHCl₃) |

### Example 11

### Methyl deoxycholate (1.22-Ester)

LiAlH(O-^{t}Bu) (332mg, 1.3 mmol,) is introduced drop-wise to a solution of Compound 1.22 (150 mg, 0.37 mmol) in THF (10 mL) under an inert atmosphere at ambient temperature. After being stirred for about 4-5 hr, the reaction mixture is quenched with Aqueous HCI (2 mL, 1 N) and the crude mixture is diluted with EtOAc (30 mL), washed with water (15 mL), saturated brine solution (10 mL) and dried over MgSO₄ (3 g). The solvent is removed under vacuum, and the crude mass is purified by column chromatography [29(W)×500(L) mm, 230-400 Mesh silica, 50 g] eluting with MeOH/CH₂Cl₂ (1 : 20) [5 mL fractions, 3 mL/min elution, monitored by TLC with p-anisaldehyde charring; R_{f} for Compound 1.22-ester = 0.42 and R_{f} for Compound 1.22 = 0.85 in MeOH/CH₂Cl₂ (1 : 9)] to afford methyl deoxycholate (Compound 1.22-ester) (110 mg, 72.8% yield) as a colorless solid. Table 14 describes the measured properties of the product.

**Table 14**

| | |
|---|---|
| ¹H NMR (500 MHz, CDCl₃) | δ = 3.97 (s, 1H), 3.65 (s, 3H), 3.63-3.59 (m, 1H), 2.39-2.33 (m, 1H), 2.25-2.19 (m, 1H), 1.88-0.97 (m, 24H), 0.95 (d, *J* = 6.0 Hz, 3H), 0.90 (s, 3H), 0.67(s, 3H) |
| ¹³C NMR (125 MHz, CDCl₃) | δ = 174.7, 73.1, 71.7, 51.4, 48.2, 47.3, 46.5, 42.1, 36.4, 36.0, 35.2, 35.1, 34.1, 33.6, 31.1, 30.9, 30.4, 28.6, 27.4, 27.1, 26.1, 23.6, 23.1, 17.3, 12.7 |
| Mass (m/z) | 407.1 [M⁺ + 1], 424.2 [M⁺ + 18] |
| IR (KBr) | 3419, 2937, 2864, 1740, 1724, 1448, 1377, 1043 cm⁻¹ |
| m.p. | 58.0-60.0 °C (under re-crystallization) |
| [α]_{D} | +36 (c = 1 in CHCl₃) |

### Example 11

### Deoxycholic Acid

A solution of LiOH (23 mg, 0.55 mmol) in H₂O (2.0 mL) is added to a solution of 1.22-ester (110 mg, 0.27 mmol) in THF (4 mL). The resulting reaction mixture is stirred for about 2-3 h at ambient temperature. Upon disappearance of the ester by TLC [R_{f} for Compound DCA = 0.35 and R_{f} for Compound 1.22-ester = 0.42 in MeOH/CH₂Cl₂ (1 : 9)], the crude reaction mixture is diluted with ethyl acetate (10 mL) and triturated with saturated brine solution to obtain a clear separation of the organic layer. The organic layer was washed with saturated NH₄Cl solution (10 mL), and dried over MgSO₄ (3.0 g). The solvent is removed under vacuum and any trace of water is removed by azetroping with toluene (3 × 5 mL) to afford deoxycholic acid (DCA) (100 mg, 94.3% yield) as a colorless solid. Table 15 describes the measured properties of the product.

**Table 15**

| | |
|---|---|
| ¹H NMR (500 MHz, DMSO) | δ = 3.77 (s, 1H), 3.38-3.33 (m, 1H), 2.20-2.15 (m, 1H), 2.08-2.02 (m, 1H), 1.92-0.90 (m, 24H), 0.84-083 (d, *J* = 5.0 Hz, 3H), 0.77 (s, 3H), 0.53 (s, 3H) |
| ¹³C NMR (125 MHz, DMSO) | δ = 175.9, 71.0, 69.9, 47.4, 46.2, 45.9, 41.6, 36.3, 35.7, 35.1, 35.0, 33.8, 32.9, 31.2, 31.2, 30.2, 28.6, 27.2, 26.9, 26.1, 23.5, 23.0, 16.9, 12.4 |
| Mass (m/z) | 392 [M⁺, not detected], 410.2 [M⁺ + 18] |
| IR | 3445, 2931, 2867, 1694, 1636, 1043 cm⁻¹ |
| m.p. | 173.2-175.5 °C (From THF/CH₂Cl₂) (Observed); 174-176 °C (Reported, Alfa Aesar) and 171-174 °C (Reported, Aldrich) |
| [α]_{D} | +50 [c = 1 in MeOH and CHCl₃ (1:1)]; +54° (c = 2 in ethanol) [Alfa Aesar] |

The yield of products for the exemplary processes described in Examples 1 through 11 are described in Table 16.

**Table 16: Overall Yield of Synthetic DCA Process**

| **Compound** | **MP (°C) (observed)** | **MP (°C) Reported** | **% Yield** | **Notes** |
|---|---|---|---|---|
| Hydrocortisone | | | | |
| **1.13** | 128.9-131.1 | 128.0-131.0 | 79.00 | Hydrogenation, side chain cleavage, and PCC oxidation |
| **1.14** | 176.6-180.5 | | Crude | K-Selectride® reduction |
| **1.15** | 156.0-158.0 | | 66.40 | Acetylation |
| **1.16** | 88.5-91.2 | | 78.80 | Wittig |
| **1.17** | | | 79.00 | Ene |
| **1.18** | 174.2-175.7 | | 95.80 | Hydrogenation (Pd/C) |
| **1.19** | 188.6-191.2 | 174.0-175.0 | 85.40 | Thionyl chloride/pyridine dehydration (2-step yield) |
| **1.20** | 185.8-188.1 | | 60.50 | Chromium trioxide allylic oxidation |
| **1.21** | 179.6-182.7 | | 90.00 | Hydrogenation (Pd/C) |
| **1.22** | 133.7-135.9 | 136.5-137.5 | 70.80 | Hydrolysis, esterification, and oxidation |
| **1.22-ester** | 58.0-60.0 | | 72.80 | LiAlH(O-*^{t}*Bu)₃ reduction |
| **DCA** | 173.2-175.5 | 174.0-176.0 | 94.33 | Hydrolysis of ester |
| | | | 7.00 | Overall yield |

### EXAMPLE 12

### 9a-Hydroxy-5β-androstan-3,17-dione (2)

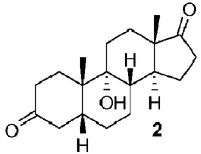

To a solution of 9α-hydroxyandrost-4-en-3,17-dione 1 (30.0 g, 99.3 mol) in DMF (150 mL) was added 10% of Pd/C (2.1 g) and the resulting slurry was hydrogenated in a Parr apparatus (60 psi) for 12 h. Upon complete disappearance of starting material, as evidenced by TLC, the crude reaction mixture was filtered through a small pad of Celite®, and the solvent was removed under vacuum to provide a colorless solid (30.0 g). This solid was combined with acetone (90 mL.) at 0 °C and the resulting slurry was stirred for 1 h. It was then filtered, washed with chilled (0 °C) acetone (30 mL) and dried under vacuum in the same filtration funnel at room temperature to afford compound 2 (26.0 g, 86%). Table 17 describes the measured properties of the product.

**Table 17**

| | |
|---|---|
| TLC | *p*-anisaldehyde charring, R_{f} for 1.1 = 0.48 and R_{f} for 1.0 = 0.30 |
| TLC mobile phase | 30% - EtOAc in DCM |
| ¹H NMR (500 MHz, CDCl₃) | δ = 2.37-2.40 (m, 1H), 2.02-.2.11 (m, 2H), 1.31-1.91 (m, 19H), 0.96 (s, 3H), 0.84 (s, 3H) |
| ¹³C NMR (125 MHz, CDCl₃) | δ = 221.0, 95.7, 80.1, 47.0, 43.6, 38.6, 38.5, 37.1, 35.9, 33.41, 32.9, 32.0, 27.9, 26.9, 21.5, 20.2, 20.0, 12.6 |
| Mass (m/z) | 305.0[M⁺ + 1], 322.0 [M⁺ + 18] |
| IR (KBr) | 3443, 2938, 1722, 1449, 1331, 1138 cm⁻¹ |
| m.p. | 213-216 °C (from DMF and acetone) |
| [α]_{D} | +116 (c = 1% in CHCl₃) |
| ELSD Purity | more than 99%, ret. time = 8.15, 9-HAD ret. time = 3.88, 5α-isomer of Cmpd 121 ret. time = 4.91 (Water symmetry 250×4.6 mm, 5um, C18), Water: ACN (40:60) |

### EXAMPLE 13

### 5β-Androst-9(11)-en-3,17-dione (3)

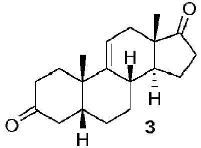

To a solution of compound 2 (26.0 g, 85.4 mmol) in DCM (520 mL) was added concentrated sulfuric acid (7.53 g, 76.8 mmol) over 15 minutes under an inert atmosphere at 10 °C. The temperature was raised to 25 °C and the resulting solution was stirred for 2 h. At this point no more starting material remained as evidenced by TLC. The reaction was quenched by the addition of 10% aqueous NaHCO₃ solution (200 mL). The layers were separated and the aqueous layer was extracted twice with DCM (2×100 mL). The organic layers were combined and washed sequentially with water (100 mL) and saturated brine solution (100 mL). The organic phase was then dried over Na₂SO₄ (75 g) and filtered. The filtrate was evaporated under vacuum to provide compound 3 (23.0 g, 94%) as an off-white solid. This product was used "as is" in the next step without further purification. Table 18 describes the measured properties of the product.

**Table 18**

| | |
|---|---|
| TLC | *p*-anisaldehyde charring, R_{f} for 1.2 = 0.76 and R_{f} for 1.1 = 0.44 |
| TLC mobile phase | 30% - EtOAc in DCM |
| ¹H NMR (500 MHz, CDCl₃) | δ = 5.61 (s , 1H ), 2.47-2.57 (m, 2H), 2.24-2.42 (m, 4H), 2.05-2.20 (m, 3H), 1.86-1.99 (m, 2H), 1.84-1.85 (d, *J* = 6 Hz 1H), 1.57-1.63 (m, 5H), 1.37-1.40 (d, *J* = 13.5 Hz, 1H) 1.25-1.28 (dd, *J* = 4.0, 13.5 Hz, 1H), 1.17 (s, 3H)0.85 (s, 3H) |
| ¹³C NMR (125 MHz, CDCl₃) | δ = 221.3, 212.8, 140.1, 118.5, 48.5, 45.9, 44.3, 43.5, 39.0, 38.0, 37.3, 36.1, 35.8, 33.3, 28.8, 26.0, 25.5, 22.5, 13.9 |
| Mass (m/z) | 287 [M⁺ + 1], 304 [M⁺ + 18] |
| I R (KBr) | 3450, 2913, 1737, 1707,1413, 1403,1207 cm⁻¹ |
| m.p. | 143.4-145.9 °C (from DCM) |
| [α]_{D} | +142 (c = 1% in CHCl₃) |
| ELSD Purity | 99.7%, Retention time = 5.04, (Inertsil ODS 3V 250 × 4.6 mm, 5um), ACN: 0.1% TFA in water (90:10) |

### EXAMPLE 14

### 3α-Hydroxy-5β-androst-9(11)-en-17-one (4)

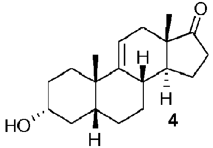

A THF solution of lithium tri-*tert*-butoxyaluminum hydride (1.0 M, 84.4 mL, 84.4 mmol) was added to a cold (-40 °C) solution of compound 3 (23.0 g, 80.4 mmol) in THF (230 mL) under an inert atmosphere. The resulting reaction mixture was stirred for 2 h. At this point the reaction was determined to be complete, as evidenced by TLC, and the reaction mixture was quenched by adding a mixture of 1 N HCI (200 mL) and ethyl acetate (230 mL). The resulting two phase mixture was separated and the aqueous layer was extracted twice with ethyl acetate (2×100 mL). The organic phases were combined and washed sequentially with water (150 mL) and saturated brine solution (100 mL). The organic phase was then dried over Na₂SO₄ (75 g) and filtered. The filtrate was evaporated under vacuum to afford compound 4 (23.0 g) as an off-white solid. The above crude product was used "as is" in the next step without purification. Table 19 describes the measured properties of the product.

**Table 19**

| | |
|---|---|
| TLC | *p*-anisaldehyde charring, R_{f} for 1.3 = 0.44 and R_{f} for 1.2 = 0.74 |
| TLC mobile phase | 30% - EtOAc in DCM (30%) |
| ¹H NMR (500 MHz, CDCl₃) | δ = 5.41-5.42 (d, *J* = 6.0 Hz, 1H), 3.65-3.66 (m, 1H), 2.43-2.48 (m, 1H), 1.98-2.18 (m, 6H), 1.74 (s, 2H), 1.48-1.56 (m, 5H), 1.377-1.45 (m, 3H), 1.18-1.28 (m, 3H), 1.08 (s, 3H), 0.80 (s, 3H) |
| ¹³C NMR (125 MHz, CDCl₃) | δ = 222.0, 140.9, 118.3, 71.9, 48.6, 45.9, 41.7, 38.8, 37.8, 36.2, 36.0, 35.7, 33.4, 31.7, 29.5, 26.5, 26.0, 22.7, 13.9 |
| Mass (m/z) | 289.0 [M⁺ + 1], 306.0 [M⁺ + 18] |
| IR (KBr) | 3463, 2960, 2871, 1729, 1366,1165,1084,1041 cm⁻¹ |
| m.p. | 165-167.5 °C (EtOAc/hexanes mixture) |
| [α]_{D} | +161 (c = 1% in CHCl₃) |
| ELSD Purity | ~ 93%, Retention time = 5.23, (Inertsil ODS 3V 250 × 4.6 mm, 5um), ACN: 0.1% TFA in water (90:10) |

### EXAMPLE 15

### (Z)-3α-Hydroxy-5β-pregna-9(11),17(20)-diene (6)

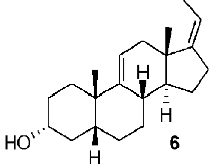

A solution of potassium *tert*-butoxide in THF (1 M, 230 mL, 231 mmol) was added drop wise to a suspension of ethyltriphenylphosphonium bromide (88.7 g, 239 mmol) in THF (150 mL) over 1 h at 25 °C. The resulting dark red colored mixture was stirred for an additional 1 h at 25 °C. A solution of compound 4 (23.0 g, 79.7 mmol) in THF (230 mL) was added slowly to the red-colored mixture at 25 °C. The resulting mixture was stirred for 3-4 h, at which point it was determined to be complete by TLC. The reaction was quenched by adding saturated aqueous NH₄Cl solution (75 mL). The phases were separated and the aqueous layer was extracted three times with EtOAc (3 × 150 mL). The organic fractions were combined, washed with saturated brine solution (100 mL), dried over Na₂SO₄ (75 g), and filtered. The filtrate was concentrated under vacuum and the crude solid was purified by column chromatography [49 mm (W) × 600 mm (L), 60-120 mesh silica, 300 g] eluting with ethyl acetate/hexanes (1:9). The fractions containing product were combined and concentrated, providing compound 6 (19.1 g, 80.0%) as a white solid. Table 20 describes the measured properties of the product.

**Table 20**

| | |
|---|---|
| TLC | *p*-anisaldehyde charring, R_{f} for 1.5 = 0.72 and R_{f} for 1.3 = 0.46 |
| TLC mobile phase | 30% - EtOAc in DCM |
| ¹H NMR (500 MHz, CDCl₃) | δ = 5.38 (s, 1H), 5.18-5.19 (d, *J* = 6.5 Hz 1H), 3.62-3.66 (m, 1H), 2.35-2.38 (d, *J* = 15 Hz, 3H), 2.23-2.25 (m, 1H), 1.97-2.07 (m, 3H), 1.64-1.75 (m, 6H), 1.32-1.55 (m, 6H), 1.17-1.24 (m, 4H), 1.06 (s, 3H), 0.79 (s, 3H) |
| ¹³C NMR (125 MHz, CDCl₃) | δ = 150.1, 140.6, 119.6, 114.2, 72.2, 53.6, 42.0, 41.9, 39.6, 38.6, 37.9, 35.7, 35.6, 31.9, 31.8, 29.5, 26.9, 26.8, 25.5, 16.9, 13.3 |
| Mass (m/z) | 301 [M⁺ + 1], 318[M⁺ + 18] |
| IR (CHCl₃) | 3304, 3033, 2925, 2863, 1449, 1368, 1040, 823cm⁻¹ |
| m.p. | 146-147.3 °C (EtOAc/hexanes mixture) |
| [α]_{D} | +84.4 (c = 1 % in CHCl₃) |
| ELSD Purity | 99.8%, Retention time = 16.07, (Inertsil ODS 3V 250 × 4.6 mm, 5um), ACN: 0.1% TFA in water (90:10) |

### EXAMPLE 16

### (Z)-3α-Acetoxy-5β-pregna-9(11),17(20)-diene (7a)

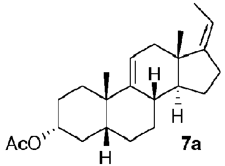

Compound 6 (19.0 g, 63 mmol) was dissolved in CH₂Cl₂ (380 mL). Triethylamine (17.6 mL, 126.6 mmol), DMAP (0.772 g, 6 mmol) and acetic anhydride (8.98 mL, 94 mmol) were added sequentially at 25 °C under a nitrogen atmosphere. The resulting solution was stirred for 2 h at 25 °C, at which point the reaction was determined by TLC to be complete. The reaction was quenched by the addition of ice-water (100 mL) and the phases were separated. The aqueous layer was extracted three times with DCM (3 × 150 mL). The organic fractions were combined and washed with saturated brine solution (100 mL), dried over anhydrous Na₂SO₄ (50 g), and filtered. The filtrate was concentrated under vacuum to afford compound 7a (22.0 g, 95% yield) as an off-white solid. Table 21 describes the measured properties of the product.

**Table 21**

| | |
|---|---|
| TLC | *p*-anisaldehyde charring, R_{f} for 1.6 = 0.5 and R_{f} for 1.5 = 0.15 |
| TLC mobile phase | 10% - EtOAc in hexanes |
| ¹H NMR (500 MHz, CDCl₃) | δ = 5.38 (s, 1H), 5.18-5.20 (d, *J* = 6.5 Hz, 1H), 4.72-4.76 (m, 1H), 2.35-2.40 (m, 3H), 2.22-2.25 (m, 1H), 2.03-2.09 (m, 3H), 2.01 (s, 3H), 1.49-1.98 (m, 10H), 1.31-1.41 (m, 2H), 1.16-1.27 (m, 3H), 1.07 (s, 3H), 0.79 (s, 3H) |
| ¹³C NMR (125 MHz, CDCl₃) | δ = 170.5, 150.0, 140.4, 119.6, 114.3, 74.7, 53.5, 42.0, 41.7, 39.6, 38.6, 35.6, 35.3, 33.8, 31.9, 29.5, 27.8, 26.7, 26.6, 25.5, 21.3, 16.9, 13.2 |
| Mass (m/z) | 342.9 [M⁺ + 1], 360 [M⁺ + 18] |
| IR(CHCl₃) | 3440, 3035, 1730, 1451, 1367, 1258, 1028cm⁻¹ |
| m.p. | 93.9-97.8 °C (EtOAc/hexanes mixture) |
| [α]_{D} | +109 (c = 1% in CHCl₃) |
| HPLC Purity | 97.62%; Retention time = 17.7, (Zorbax SB, C18; 250 × 4.6 mm, 5um), ACN: 0.1% TFA in water (90:10) |

### EXAMPLE 17

### (E)-Methyl 3α-acetoxy-5β-chol-9(11), 16, 22-trien-24-oate (8a)

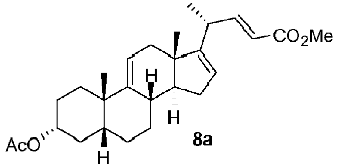

Ethyl aluminum dichloride (104.5 mL, 192 mmol, 1.8 M in toluene) was added to a solution of methyl propiolate (13.58 mL, 153 mmol) in DCM (100 mL) at 0 °C under inert atmosphere. The resulting solution was stirred for 15 min and then compound 7a (22 g, 64.3 mmol) was added. After stirring for an additional 20 min at 0 °C, the temperature was raised to 25 °C and held there for a further 18 h. At this point the reaction was determined to be complete by TLC, and the mixture was poured into cold (0 °C) water (200 mL). The phases were separated and the aqueous layer was extracted with DCM (150 mL). The organic layers were combined and washed sequentially with water (200 mL) and saturated brine solution (100mL). It was then dried over anhydrous Na₂SO₄ (40 g) and filtered. The filtrate was concentrated under vacuum and the resulting solid was purified by slurring in methanol (280 mL) to provide compound 8a (17.5 g 68%) as a white solid. Table 22 describes the measured properties of the product.

**Table 22**

| | |
|---|---|
| TLC | *p*-anisaldehyde charring, R_{f} for 1.7a = 0.32 and R_{f} for 1.6a = 0.5 |
| TLC mobile phase | 10% - EtOAc in hexanes |
| ¹H NMR (500 MHz, CDCl₃) | δ = 6.92-6.926 (q, *J* = 7.5, 15.5 Hz, 1H), 5.80-5.83 ( d, *J* = 16 Hz, 1H), 5.37-5.43 (m, 2H), 4.73-4.75 (m, 1H), 3.73 (s, 3H), 3.02-3.04 (t, *J* = 6.5 Hz, 1H), 2.15-2.23 (m, 3H), 2.05-2.08 (m, 3H), 2.01 (s, 3H), 1.48-1.99 (m, 8H), 1.24-1.34 (m, 2H), 1.20-1.21 (d, *J* = 5 Hz, 3H), 1.11-1.17 (m, 1H), 1.07 (s, 3H), 0.67 (s, 3H) |
| ¹³C NMR (125 MHz, CDCl₃) | δ =170.5, 167.2, 155.0, 153.7, 141.6, 124.0, 118.8, 118.7, 74.6, 53.9, 51.3, 45.7, 41.7, 38.8, 37.1, 35.5, 35.3, 34.6, 33.7, 31.8, 29.5, 27.7, 26.5, 26.5, 21.3, 19.7, 15.7 |
| Mass (m/z) | 444.0 [M⁺ + 18] |
| IR (KBr) | 3443, 3030, 2930, 1719, 1650, 1247, 1359, 1032, 1170 cm⁻¹ |
| m.p. | 114-116 °C (from methanol) |
| [α]_{D} | +102 (c = 1% in CHCl₃) |
| ELSD Purity | 99.7%, Retention time = 19.57, (Inertsil ODS 3V 250 × 4.6 mm, 5um), ACN: 0.1% TFA in water (90:10) |

### EXAMPLE 18

### Methyl 3α-acetoxy-5β-chol-9(11)-en-24-oate (9a)

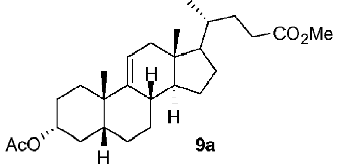

To a solution of compound 8a (17.5 g, 41 mmol) in EtOAc (350 mL) was added PtO₂ (4.37 g), and the resulting slurry was hydrogenated in a Parr apparatus (70 psi) for 14-16 h. At this point the reaction was determined to be complete by TLC. The mixture was filtered through a small plug of Celite® and the solvent was removed under vacuum, affording compound 9a (17.0 g, 96.0%) as a white solid. The above product was used in the next step without further purification. Table 23 describes the measured properties of the product.

**Table 23**

| | |
|---|---|
| TLC | *p*-anisaldehyde charring, R_{f} for 1.8a = 0.32 and R_{f} for 1.7a = 0.30 |
| TLC mobile phase | 10% - EtOAc in hexanes |
| ¹H NMR (500 MHz, CDCl₃) | δ = 5.31 (s, 1H), 4.73 (m, 1H), 3.66 (s, 3H), 2.03-2.37 (m, 7H), 2.01 (s, 3H), 1.09-1.98 (m, 18H), 1.06 (s, 3H), 0.91-0.92 (d, *J* = 6.0 Hz, 3H), 0.59 (s, 3H) |
| ¹³C NMR (125 MHz, CDCl₃) | δ = 174.6, 170.5, 139.8, 119.5, 74.8, 56.0, 53.3, 51.4, 41.9, 41.7, 40.9, 38.5, 36.4, 35.4, 35.2, 33.8, 31.0, 30.9, 29.5, 28.2, 27.8, 26.8, 26.7, 25.2, 21.4, 17.9, 11.5 |
| Mass (m/z) | 448.2 [M⁺ + 18] |
| IR (KBr) | 3435, 3039, 2941, 1729, 1448, 1435, 1252, 1022 cm⁻¹ |
| m.p. | 122.1-123.9 °C (from EtOAc) |
| [α]_{D} | +56 (*c* = 1% in CHCl₃) |
| ELSD Purity | 97.7%: Retention time = 14.57 (ZORBAX SB C-18 150 × 4.6 mm, 5um, ACN: 0.1% TFA in water (90:10) |

### EXAMPLE 19

### Methyl 3α-acetoxy-5β-chol-9(11), 16-dien-24-oate (8b)

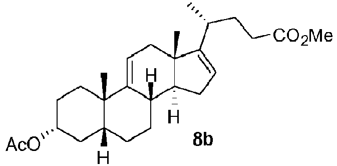

Ethyl aluminum dichloride (14.2 mL, 25 mmol, 1.8M in toluene) was added to a solution of methyl acrylate (1.89 mL, 20 mmol) in DCM (60 mL) at 0°C under inert atmosphere. The resulting solution was stirred for 15 min and then compound 7a (3 g, 8.7 mmol) was added. After stirring for an additional 20 min at 0 °C, the temperature was raised to 25 °C and held there for a further 18 h. At this point the reaction was determined to be complete by TLC, then the mixture was poured into cold (0 °C) water (60 mL). The phases were separated and the aqueous layer was extracted with DCM (60 mL). The organic layers were combined and washed sequentially with water (50 mL) and saturated brine solution (1 00mL). It was then dried over anhydrous Na₂SO₄ (5 g) and filtered. The filtrate was concentrated under vacuum, providing compound **8b** (2.6 g, 70%). Table 24 describes the measured properties of the product.

**Table 24**

| | |
|---|---|
| TLC mobile phase | 10% - EtOAc in hexanes |
| ¹H NMR (500 MHz, CDCl₃) | δ =5.34-5.43 (m, 2H), 4.73-4.75 (m, 1H), 3.73 (s, 3H), 2.15 - 2.34 (m, 6H), 2.05-2.08 (m, 3H), 2.01 (s, 3H), 1.48-1.99 (m, 9H), 1.24-1.34 (m, 3H), 1.20-1.21 (d, *J* = 5 Hz, 3H), 1.11-1.17 (m, 1H), 1.07 (s, 3H), 0.67 (s, 3H) |
| ELSD Purity | 93.9%, ret. time = 4.55, (Water symmetry shield 250×4.6mm 5µ), ACN:100% |

### EXAMPLE 20

### Methyl 3α-acetoxy-5β-chol-9(11)-en-24-oate (9a)

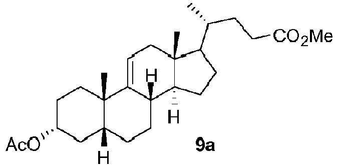

To a solution of compound 8a (3 g, 7 mmol) in EtOAc (60 mL) was added 10% Pd/C (300mg, 10% wt/wt), and the resulting slurry was hydrogenated in a Parr apparatus (70 psi) for 14-16 h. At this point the reaction was determined to be complete by TLC (10% ethyl acetate in hexanes). The mixture was filtered through a small plug of Celite® and the solvent was removed under vacuum, affording compound 9a (2.6g, 86%) as a white solid. Table 25 describes the measured properties of the product.

**Table 25**

| | |
|---|---|
| ¹H NMR (500 MHz, CDCl₃) | δ = 5.31 (s, 1H), 4.73(m, 1H), 3.66 (s, 3H), 2.37-2.03 (m, 7H), 2.01 (s, 3H), 1.98-1.09(m, 18H), 1.06 (s, 3H), 0.92-0.91 (d, *J* = 6.0 Hz, 3H), 0.59 (s, 3H) |
| ELSD Purity | 95.9%, ret. time = 4.75, (Water symmetry shield 250×4.6 mm 5µ), ACN:Water (60:40) |

### EXAMPLE 21

### Methyl 3α-acetoxy-5β-chol-9(11)-en-12-one-24-oate (10a)

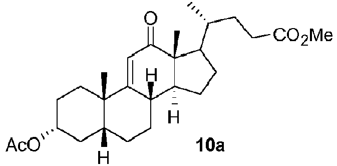

CrO₃ (17.0 g, 170 mmol) was added to a solution of compound 9a (17 g, 39.5 mmol) in AcOH (270 mL). The resulting mixture was heated at 50 °C for 24-36 h. Upon complete disappearance of the starting material by TLC, the solvent was evaporated under vacuum and the crude material was dissolved in ethyl acetate (400 mL) and water (200 mL). The two phases were separated and the organic layer was washed twice with water (2 × 100 mL) and then once with saturated brine solution (100 mL). The organic phase was dried over anhydrous Na₂SO₄ (40 g) and filtered. The filtrate was concentrated under vacuum and the resulting solid was purified by column chromatography [49 mm (W) × 600 mm (L), 60-120 mesh silica, 120 g], eluting with ethyl acetate/hexane (1 : 5) [10 mL fractions, 3 mL/min elution, monitored by TLC and detected with UV light (254 nm) lamp]. The product-containing fractions were combined and concentrated under vacuum to afford compound 10a (8.8 g, 50% yield) as a white solid. Table 26 describes the measured properties of the product.

**Table 26**

| | |
|---|---|
| TLC | *p*-anisaldehyde charring, R_{f} for 1.9a = 0.28 and R_{f} for 18a = 0.52 |
| TLC mobile phase | 20% - EtOAc in hexanes |
| ¹H NMR (500 MHz, CDCl₃) | δ = 5.71 (s, 1H), 4.71-4.75 (m, 1H), 3.66 (s, 3H), 2.37-2.42 (m, 3H), 2.02-2.31 (m, 2H), 2.0 (s, 3H), 1.67-1.98 (m, 9H), 1.24-1.56 (m, 9H), 1.19 (s, 3H), 1.01-1.02 (d, *J* = 6.5 Hz, 3H), 0.90 (s, 3H) |
| ¹³C NMR (500 MHz, CDCl₃) | δ = 204.9, 174.5, 170.4, 163.8, 123.6, 73.7, 53.4, 53.0, 51.3, 47.2, 41.7, 39.8, 37.7, 35.2, 35.0, 33.9, 31.4, 30.5, 29.6, 27.6, 27.3, 26.4, 26.1, 24.1, 21.2, 19.4, 10.6 |
| Mass (m/z) | 445.0 [M⁺ + 1], 462.0 [M⁺ + 18] |
| IR | 3437, 3045, 2946, 2870, 1729, 1680, 1252, 1168, 1020, cm⁻¹ |
| m.p. | 137-139 °C (from EtOAc/hexanes mixture) |
| [α]_{D} | +93 (c = 1 % in CHCl₃) |
| ELSD Purity | 94.6%: Retention time = 8.68 (Inertsil ODS 3V, 250 × 4.6 mm, 5um, ACN: Water (60:40) |

### EXAMPLE 22

### Methyl 3α-acetoxy-5β-cholan-12-one-24-oate (11 a)

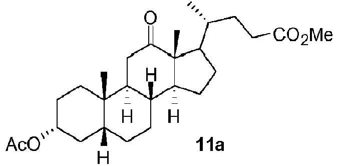

10% Pd/C (900 mg) was added to a solution of compound 10a (2.0 g, 4.5 mmol) in EtOAc (150 mL) and the resulting slurry was hydrogenated in a Parr apparatus (50 psi) at 50 °C for 16 h. At this point the reaction was determined to be complete by TLC. The mixture was filtered through a small plug of Celite® and the solvent was removed under vacuum, providing compound 11 a (1.6 g, 80% yield) as a white solid. Table 27 describes the measured properties of the product.

**Table 27**

| | |
|---|---|
| TLC | *p*-anisaldehyde charring, R_{f} for 2.0 = 0.36 and R_{f} for 1.9 = 0.32 |
| TLC mobile phase | 20% - EtOAc in hexanes |
| ¹H NMR (500 MHz, CDCl₃) | δ = 4.67-4.71 (m, 1H), 3.66 (s, 3H), 2.45-2.50 (*t, J* = 15 Hz, 2H), 2.22-2.40 (m, 1H), 2.01 (s, 3H), 1.69-1.96 (m, 9H), 1.55 (s, 4H), 1.25-1.50 (m, 8H), 1.07-1.19 (m, 2H), 1.01 (s, 6H), 0.84-0.85 (d, *J* = 7.0 Hz, 3H) |
| ¹³C NMR (125 MHz, CDCl₃) | δ = 214.4, 174.5, 170.4, 73.6, 58.5, 57.4, 51.3, 46.4, 43.9, 41.2, 38.0, 35.6, 35.5, 35.2, 34.8, 32.0, 31.2, 30.4, 27.4, 26.8, 26.2, 25.9, 24.2, 22.6, 21.2, 18.5, 11.6 |
| Mass (m/z) | 447.0 [M⁺ + 1], 464.0 [M⁺ + 18] |
| IR (KBr) | 3445, 2953, 2868, 1731, 1698, 1257, 1029 cm⁻¹ |
| m.p. | 142.2-144.4 °C (from EtOAc/hexanes mixture) |
| [α]_{D} | +92 (c = 1 % in CHCl₃) |
| ELSD Purity | 96.6%: Retention time = 9.93 (Inertsil ODS 3V, 250 × 4.6 mm, 5um, ACN: 0.1% TFA in water (90:10) |

### EXAMPLE 23

### Methyl 3α-acetoxy-12a-hydroxy-5β-cholan-24-oate (12a)

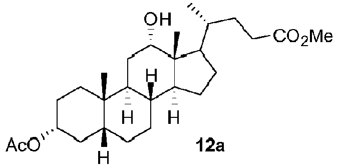

A THF solution of lithium tri-*tert*-butoxyaluminum hydride (1 M, 22.4 mL, 22.4 mmol) was added drop wise to a solution of compound 11 a (2.5 g, 5.6 mmol) in THF (25 mL) at ambient temperature. After stirring for an additional 4-5 h, the reaction was determined to be complete by TLC. The reaction was quenched by adding aqueous HCl (1 M, 10 mL) and the mixture was diluted with EtOAc (30 mL). The phases were separated and the organic phase was washed sequentially with water (15 mL) and saturated brine solution (10 mL). The organic phase was then dried over anhydrous Na₂SO₄ (3 g) and filtered. The filtrate was concentrated under vacuum and the resulting solid was purified by column chromatography [29 mm (W) × 500 mm (L), 60-120 mesh silica, 50 g], eluting with EtOAc/hexane (2:8) [5 mL fractions, monitored by TLC with p-anisaldehyde charring]. The fractions containing the product were combined and concentrated under vacuum to provide compound 12a (2.3 g, 91 %) as a white solid. Table 28 describes the measured properties of the product.

**Table 28**

| | |
|---|---|
| TLC | *p*-anisaldehyde charring, R_{f} for 2.1 a = 0.45 and R_{f} for 2.0a = 0.55 |
| TLC mobile phase | 30% - EtOAc in hexanes |
| ¹H NMR (500 MHz, CDCl₃) | δ = 4.68-4.73 (m, 1H), 3.98 (s, 1H), 3.66 (s, 3H), 2.34-2.40 (m, 1H), 2.21-2.26 (m, 1H), 2.01 (s, 3H), 1.75-1.89 (m, 6H), 1.39-1.68 (m, 16H), 1.00-1.38 (m, 3H), 0.96-0.97 (d, *J* = 5.5 Hz, 3H), 0.93 (s, 3H), 0.68 (s, 3H) |
| ¹³C NMR (125 MHz, CDCl₃) | δ = 174.5, 170.5, 74.1, 72.9, 51.3, 48.1, 47.2, 46.4, 41.7, 35.8, 34.9, 34.7, 34.0, 33.5, 32.0, 30.9, 30.8, 28.6, 27.3, 26.8, 26.3, 25.9, 23.4, 22.9, 21.3, 17.2, 12.6 |
| Mass (m/z) | 449.0 [M⁺ + 1], 466.0 [M⁺ + 18] |
| IR (KBr) | 3621, 2938, 2866, 1742, 1730, 1262, 1162, 1041, cm⁻¹ |
| m.p. | 104.2-107.7 °C (from EtOAc) |
| [α]_{D} | +56 (c = 1 % in CHCl₃) |
| ELSD Purity | 97.0%: Retention time = 12.75 (Inertsil ODS 3V, 250 × 4.6 mm, 5um,. ACN: Water (60:40) |

### EXAMPLE 24

### Deoxycholic acid (DCA)

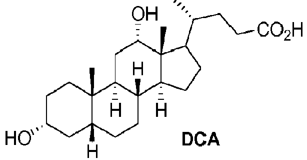

A solution of LiOH (187 mg, 4.4 mmol) in H₂O (2.0 mL) was added to a solution of compound 12a (500 mg, 1.11 mmol) in THF (8 mL) and MeOH (8 mL). The resulting mixture was stirred for 3-4 h at 50 °C. Upon complete disappearance of the starting material by TLC, the reaction mixture was concentrated under vacuum. A mixture of water (10 mL) and 3 N HCI (1 mL) were combined and cooled to 0 °C and then added to the crude product. After stirring for 1 h at 0 °C, the precipitated solids were filtered and then washed with water (10 mL) and hexane (20 mL). Drying under vacuum at room temperature provided deoxycholic acid (DCA, 400 mg, 91% yield) as a white solid. Table 29 describes the measured properties of the product.

**Table 29**

| | |
|---|---|
| TLC | *p*-anisaldehyde charring, R_{f} for DCA = 0.32 and R_{f} for 2.1 a = 0.82 |
| TLC mobile phase | 10% - Methanol in DCM |
| ¹H NMR (500 MHz, CDCl₃) | δ = 11.92 (s, 1H), 4.44 (s, 1H), 4.19 (s, 1H), 3.77 (s, 1H), 3.35-3.36 (m, 1H), 2.19-2.21 (m, 1 H), 2.08-2.10 (m, 1H), 1.73-1.80 (m, 4H), 1.43-1.63 (m, 6H), 1.15-1.35 (m, 12H), 0.98-1.05 (m, 2H), 0.89-0.90 (d, *J* = 6.0 Hz, 3H), 0.83 (s, 3H), 0.58 (s, 3H) |
| ¹³C NMR (125 MHz, DMSO) | δ =174.8, 71.0, 69.9, 47.4, 46.1, 46.0, 41.6, 36.3, 35.6, 35.1, 34.9, 33.8, 32.9, 30.8, 30.7, 30.2, 28.6, 27.1, 27.0, 26.1, 23.5, 23.0, 16.9, 12.4 |
| Mass (m/z) | 393 [M⁺, + 1] |
| IR (KBr) | 3363, 2933, 2863, 1694, 1453, 1372, 1042, cm⁻¹ |
| m.p. | 171.4-173.6 °C (from ethanol); 174-176 °C (Alfa Aesar) and 171-174 °C (Aldrich) |
| [α]_{D} | +47 (c = 1 % in EtOH ), +54° (c = 2% in ethanol) [Alfa Aesar] |
| ELSD Purity | 99.7%: Retention time = 5.25 (Inertsil ODS 3V, 250 × 4.6 mm, 5um, ACN: 0.1% TFA in water (90:10) |

### REFERENCE EXAMPLE 25

Primary human adipocytes were incubated with varying concentrations of synthetic sodium deoxycholate synthesized using 9-HAD as starting material or bovine-derived sodium deoxycholate obtained from Sigma as described below.

### Materials

Adipocytes (Zen-Bio cat# SA-1096)
96 well plates (US Scientific cat# cellstar no. 655180)
Serum-free RPMI medium (Mediatech cat# 17-105-CV)
Sodium deoxycholate (DC) (Sigma cat# D6750)
Synthetic Sodium glycodeoxycholate (Kythera)
PBS (1x)
MTS assay kit (Promega cat# G3580)

Adipocytes arrived differentiated and at a density of 13,000 cells per well in a 96 well plate. Two plates were received and each treated with the same samples. Cells were incubated for 24 hours at 37 °C with 5% CO₂. A 1 % stock solution of each bile acid (synthetic and non-synthetic DCA) was made by dissolving 20 mg into 2 mL media (serum-free). Using the 1% stock solution, the following 11 solutions were prepared by dilution: 0.005%, 0.01%, 0.015%, 0.02%, 0.025%, 0.03%, 0.035%, 0.04%, 0.05%, 0.06%, and 0.1% , as well as 0% (media only).

Cells were washed 2x with 150 µL of room temperature 1x PBS (phosphate buffered saline). Media and then PBS were removed from the wells in a 96 well plate by turning the plate upside down and decanting the liquid into a container. After the last PBS wash, 80 µL of sample was added per well. Each concentration of a specific bile acid was added to 8 wells and incubated for 1 hour at 37 °C with 5% CO₂. Plates were then removed from incubator and solution was decanted. A 100 µL solution of diluted (40 µL in 1 mL of RPMI) MTS reagent (3-(4,5-dimethylthiazol-2-yl)-5-(3-carboxymethoxyphenyl)-2-(4-sulfophenyl)-2H-tetrazolium, inner salt) was added directly to each well. Plates were incubated at 37 °C with 5% CO₂ until control (no bile acid) wells changed color to orange-brown and then loaded onto a spectrophotometer that analyzes 96 well plates. Samples were run at 490 nm wavelength setting.

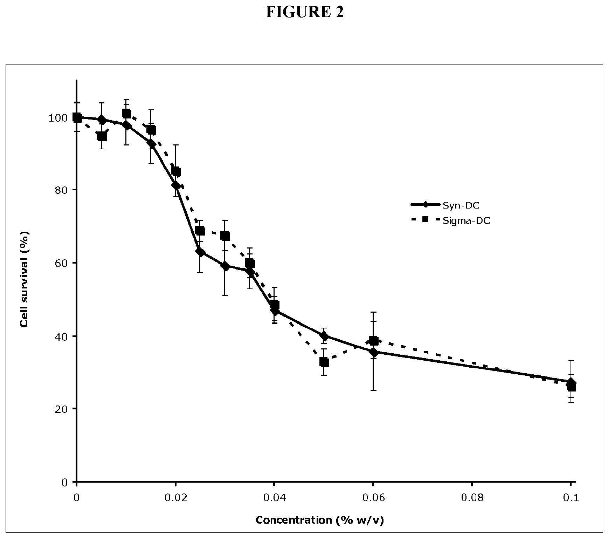

Cell viability was assessed using a colorimetric assay (MTS) kit from Promega. The results show a dose-dependent decrease in cell survival upon treatment with either syn-NaDC or Sigma-NaDC (see **Figure 2**). Both molecules demonstrated similar cytolytic behavior in this experiment, indicating that synthetic-NaDC and bovine-derived Sigma-NaDC are functionally identical in terms of their ability to kill fat cells.

## Claims

1. A method of preparing a compound of formula 12: 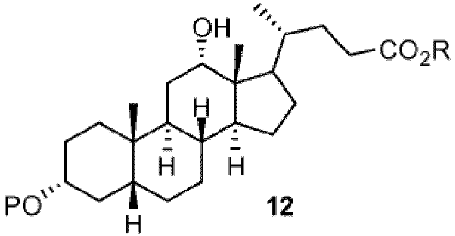 wherein P is a protecting group and R is alkyl, said method comprising reacting a compound of formula 11: 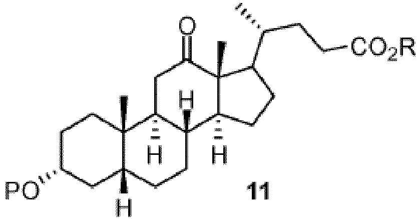 wherein P and R are defined as in formula **12**, with LiAl(OtBu)₃H to provide a compound of formula 12.

2. The method of claim 1, further comprising exposing the compound of formula 12 to deprotection conditions to form an ester thereof.

3. The method of claim 2, further comprising exposing said ester to suitable hydrolysis conditions to form deoxycholic acid: 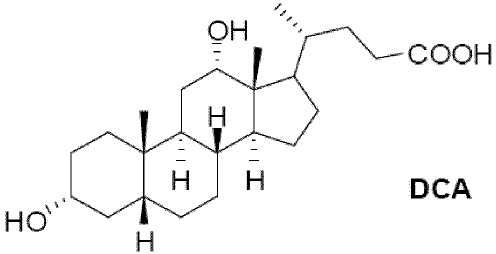 or a pharmaceutically acceptable salt thereof.

## Patentansprüche

1. Verfahren zur Herstellung einer Verbindung der Formel 12: 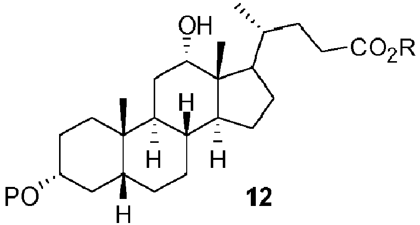 worin P eine Schutzgruppe ist und R Alkyl ist und das Verfahren das Umsetzen einer Verbindung der Formel 11: 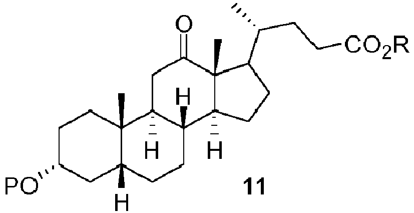 worin P und R wie in Formel 12 definiert sind, mit LiAl(OtBu)₃H umfasst, so dass eine Verbindung der Formel 12 bereitgestellt wird.

2. Verfahren gemäß Anspruch 1, welches ferner umfasst, dass die Verbindung der Formel 12 Entschützungsbedingungen ausgesetzt wird, so dass sich ein Ester davon bildet.

3. Verfahren gemäß Anspruch 2, welches ferner umfasst, dass der Esters geeigneten Hydrolysebedingungen ausgesetzt wird, so dass sich Deoxycholsäure bildet: 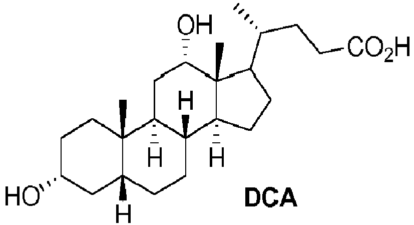 oder ein pharmazeutisch annehmbares Salz davon.

## Revendications

1. Procédé de préparation d'un composé de formule **12** : 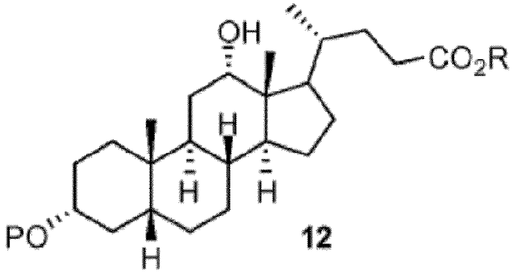 où P représente un groupement protecteur et R représente un alkyle, ledit procédé comprenant la mise en réaction d'un composé de formule **11** : 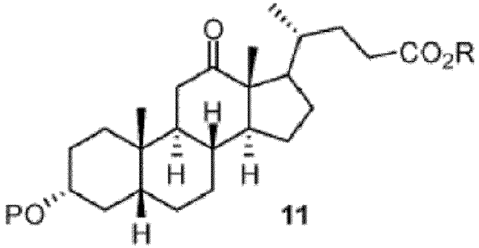 où P et R sont tels que définis dans la formule **12**, avec LiAl(OtBu)₃H pour fournir un composé de formule **12**.

2. Procédé selon la revendication 1, comprenant en outre l'exposition du composé de formule **12** à des conditions de déprotection pour produire un ester de celui-ci.

3. Procédé selon la revendication 2, comprenant en outre l'exposition dudit ester à des conditions d'hydrolyse appropriées pour produire l'acide désoxycholique : 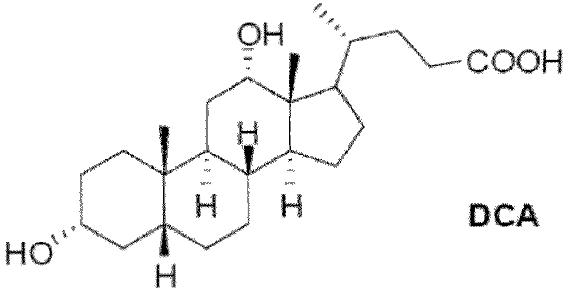 ou un sel pharmaceutiquement acceptable de celui-ci.
